# FASCICULE DE BREVET EUROPEEN

(11) **EP 0 404 689 B1**
(45) Date de publication et mention de la délivrance du brevet: **04.10.1995**
(21) Numéro de dépôt: 90401777.9
(22) Date de dépôt: 22.06.1990
(51) Int. Cl.: A61F 2/14, A61F 9/00

(54) **Lentille pour épikératophakie et kératotome notamment destiné à la réalisation d'une incision de réception d'une telle lentille**
Linse für die Epikeratophakie und Keratotom zum Ausführen eines Schnittes für die Implantation einer solchen Linse
Lens for epikeratophakia and keratotome intended for the creation of an incision for implantation of such a lens

(30) Priorité: 23.06.1989 FR 8908377
(43) Date de publication de la demande: 27.12.1990
(73) Titulaire: Hanna, Khalil, F-75007 Paris (FR)
(72) Inventeur: Hanna, Khalil, F-75007 Paris (FR)
(74) Mandataire: Martin, Jean-Jacques

(56) Documents cités:
- EP-A- 0 047 190
- EP-A- 0 308 077
- FR-A- 2 627 078
- US-A- 4 077 411
- US-A- 4 423 728
- US-A- 4 810 082

## Description

La présente invention concerne une lentille pour épikératophakie, ainsi qu'un kératotome notamment destiné à la réalisation d'une incision de réception d'une telle lentille.

L'épikératophakie est une technique de correction d'amétropie d'un globe oculaire, consistant à fixer sur la cornée de celui-ci, par l'extérieur, une lentille correctrice jusqu'à présent obtenue par prélèvement d'une cornée sur un globe donneur, puis usinage de cette cornée pour y former :
- une zone optique présentant une forme lenticulaire d'axe optique déterminé et délimitée par des faces respectivement antérieure, convexe, et postérieure, concave, de géométries respectives déterminées d'une part en fonction de la possibilité d'appliquer intimement la face postérieure de la zone optique contre la cornée du globe à corriger ou globe receveur, notamment avec coïncidence mutuelle de l'axe optique et de l'axe visuel du globe receveur, et d'autre part en fonction de caractéristiques optiques prédéterminées en fonction de la correction a apporter à ce globe, et
- une zone d'ancrage présentant une forme annulaire de révolution autour de l'axe optique et bordant la zone optique dans le sens d'un éloignement par rapport à l'axe optique, pour s'insérer dans une incision annulaire de la cornée du globe receveur.

Dans les techniques actuellement connues, la zone d'ancrage présente à la périphérie de la lentille une épaisseur importante et son insertion dans l'incision de la cornée du globe receveur nécessite une incision profonde parallèlement à l'axe visuel et ensuite une dissection profonde parallèlement à la surface de la cornée, ce qui est traumatisant et, en outre, n'assure pas un ancrage stable de la lentille sans suture sauf si la zone d'ancrage est grande ; en outre, l'épaisseur importante de la zone d'ancrage à la périphérie de la lentille provoque, après son insertion dans la cornée, un bourrelet en surépaisseur par rapport a la zone optique et quel que soit le soin apporté à la réalisation de cette lentille, c'est-à-dire au taillage de la cornée prélevée sur le globe donneur, il est pratiquement impossible d'obtenir une transition régulière entre la lentille et la cornée du globe receveur, en surface comme en profondeur, autour de la lentille ; de plus, en surface, l'épithélium peut augmenter d'épaisseur et modifier la réfraction, ce qui rend cette technique peu précise, de même que la distorsion mécanique que peut subir la lentille à l'insertion.

Dans une variante, décrite dans FR-A-2 627 078, qui sert de base à la subdivision de la revendication 1 en préambule et partie caractérisante, la zone d'ancrage de la lentille, alors réalisée en matériau polymère, n'est pas destinée à s'insérer dans une incision annulaire de la cornée du globe receveur, mais simplement à être accolée extérieurement et fixée, notamment par suture, à celle-ci. Egalement dans le cas de cette variante, l'ancrage de la lentille à la cornée manque de stabilité et la zone d'ancrage présente une épaisseur importante qui entraîne une irrégularité de transition entre la lentille et la cornée du globe receveur et une perturbation de la croissance de l'épithélium.

Le but de la présente invention est de perfectionner la technique connue d'épikératophakie, notamment en proposant une conformation plus satisfaisante de la zone d'ancrage de la lentille afin d'améliorer la transition avec la cornée notamment en surface et de réduire la distorsion de la lentille, que la lentille soit réalisée par taillage d'une cornée provenant d'un globe donneur ou directement par moulage puis usinage d'un polymère ou copolymère souple, bio-compatible, naturel ou de synthèse, ainsi qu'un kératotome particulièrement adapté à la réalisation d'une incision propre à recevoir une telle zone d'ancrage.

La lentille pour épikératophakie selon l'invention, comprenant, comme la lentille décrite dans FR-A-2 627 078 :
- une zone optique présentant une forme lenticulaire d'axe optique déterminé et délimitée par des faces respectivement antérieure, convexe, et postérieure, concave, de géométrie respective déterminée d'une part en fonction de la possibilité d'appliquer intimement la face postérieure de la zone optique contre une cornée et d'autre part en fonction de caractéristiques optiques prédéterminées, et
- une zone d'ancrage présentant une forme annulaire de révolution autour de l'axe optique et bordant la zone optique dans le sens d'un éloignement par rapport à l'axe optique, cette zone d'ancrage étant notamment délimitée par une face postérieure qui se raccorde à la face postérieure de la zone optique et forme une saillie par rapport à la face postérieure de la zone optique et par une face antérieure qui se raccorde à la face antérieure de la zone optique et est disposée en retrait par rapport à la face antérieure de la zone optique,
se caractérise en ce que les faces respectivement antérieure et postérieure de la zone d'ancrage convergent mutuellement dans le sens d'un éloignement par rapport à l'axe optique, de façon à déterminer un amincissement progressif de la zone d'ancrage à partir de la zone optique et jusqu'à un bord périphérique de la lentille, pour permettre d'insérer la zone d'ancrage dans une incision annulaire de la cornée.

Typiquement, en référence à un plan perpendiculaire à l'axe optique, la face antérieure de la zone d'ancrage présente une inclinaison sensiblement inférieure à 90°, et de préférence comprise entre 50° environ et 55° environ, alors que la face postérieure de la zone d'ancrage présente une inclinaison de préférence comprise entre 40° environ et 45° environ, et notamment de 42°.

De préférence, les faces antérieure et postérieure de la zone d'ancrage présentent approximativement la même forme et les mêmes dimensions lorsqu'elles sont vues en coupe par un quelconque demi-plan limité par l'axe optique.

La zone d'ancrage ainsi conçue forme, autour de la zone optique, un biseau propre à s'insérer dans une incision pratiquée, sous forme d'une fente annulaire d'orientation appropriée, dans la cornée du globe receveur en s'encastrant avec précision derrière une lame de cornée dégagée par cette incision, laquelle lame épouse étroitement la face antérieure de la zone d'ancrage par l'un des flancs de l'incision alors que l'autre flanc de cette incision épouse étroitement la face postérieure de la zone d'ancrage dès lors que l'incision a été correctement dimensionnée, d'une façon aisément déterminable en fonction des caractéristiques géométriques de la lentille ; cette insertion peut être facilitée par l'aménagement, dans la zone d'ancrage, d'au moins une entaille débouchant dans le bord périphérique de la lentille et dans des zones des faces antérieure et postérieure de la zone d'ancrage voisines du bord périphérique de la lentille, ce qui permet d'insérer la zone d'ancrage dans l'incision par un mouvement comparable à un mouvement de vissage.

Le fait que la zone d'ancrage s'insère étroitement dans l'incision permet une meilleure régularité de surface et évite toute dépression dans la transition entre la cornée du globe receveur et la lentille, ce qui évite la formation ultérieure de tout bourrelet résultant d'une augmentation de l'épaisseur d'épithélium.

De plus, l'incision nécessaire à la mise en place de la zone d'ancrage de la lentille conforme à la présente invention est peu traumatisante en elle-même du fait qu'elle n'entraîne pas d'enlèvement de matière de la cornée et que, en raison d'une inclinaison générale, par rapport à l'axe visuel du globe receveur, adaptée à une inclinaison générale de la zone d'ancrage par rapport à l'axe optique de la zone optique de la lentille, elle s'enfonce dans la cornée dans le sens d'un éloignement par rapport à l'axe visuel, c'est-à-dire vers des zones d'épaisseur relativement plus importante de la cornée ; l'insertion, par la suite, de la zone d'ancrage de la lentille dans cette incision n'implique en elle-même que des déformations peu traumatisantes de la cornée si bien que l'utilisation d'une lentille conforme à la présente invention offre un maximum de garanties quant à une implantation efficace et stable dans le temps.

Pour des raisons de facilité de réalisation de l'incision, il est préférable que les faces antérieure et postérieure de la zone d'ancrage soient tronconiques de révolution autour de l'axe optique, mais d'autres formes pourraient également être admises, telles que des formes approximativement tronconiques s'arrondissant au raccordement avec la face antérieure ou postérieure, respectivement, de la zone optique pour assurer une transition douce.

De même, bien qu'il soit préférable, pour des raisons géométriques, que les faces antérieure et postérieure de la zone d'ancrage se raccordent d'une part mutuellement et d'autre part, respectivement, aux faces antérieure et postérieure de la zone optique de façon directe, on peut admettre pour des raisons pratiques que ces raccordements s'effectuent respectivement par l'intermédiaire d'une face périphérique de révolution autour de l'axe optique, et par exemple cylindrique, par un décrochement périphérique de la face antérieure de la zone optique, de révolution autour de l'axe optique et par exemple cylindrique, et par un épaulement périphérique de la face postérieure de la zone optique, de révolution autour de l'axe optique et par exemple cylindrique. Le fait d'admettre un raccordement mutuel des faces antérieure et postérieure de la zone d'ancrage par l'intermédiaire d'une face périphérique de révolution autour de l'axe optique permet de faciliter la réalisation de la lentille et d'éviter un endommagement de la périphérie de celle-ci lors des manipulations ; le fait d'admettre un épaulement au raccordement de la face postérieure de la zone optique à la face postérieure de la zone d'ancrage et, conjointement ou non, un décrochement au raccordement de la face antérieure de la zone optique à la face antérieure de la zone d'ancrage peut faciliter la conception et la fabrication de la lentille lorsque, respectivement, la face postérieure ou antérieure de la zone optique présente une forme différente d'une forme de révolution autour de l'axe optique, c'est-à-dire en pratique une forme torique en vue de la correction d'un astigmatisme du globe receveur, mais on peut également choisir un tel mode de raccordement en dehors d'un tel cas dans la mesure où il est également lié à une plus grande facilité et à une plus grande précision d'incision de la cornée du globe receveur en vue de l'insertion de la zone d'ancrage de la lentille.

En effet, il est plus facile et plus précis de commencer l'incision de la cornée du globe receveur par une pénétration d'un couteau parallèlement à l'axe visuel pour poursuivre ensuite l'incision en oblique par rapport à cet axe visuel, que d'agir dès l'origine sur la cornée du globe receveur en oblique par rapport à l'axe visuel. On comprend aisément que, dans cet esprit, l'épaulement périphérique de la face postérieure de la zone optique de la lentille et le décrochement périphérique de la face postérieure de cette zone optique sont destinés à épouser étroitement les zones des flancs de l'incision correspondant au départ de celle-ci, parallèle à l'axe visuel avec lequel on fait coïncider l'axe optique de la zone optique lors de la mise en place de la lentille selon l'invention.

Toutefois, on préfère limiter au minimum nécessaire les dimensions de ce départ de l'incision parallèlement à l'axe visuel, c'est-à-dire les dimensions qu'il faut donner parallèlement à l'axe optique au décrochement de la face antérieure de la zone optique de la lentille et à l'épaulement de la face postérieure de celle-ci, de même qu'il convient de limiter les dimensions que présente parallèlement à l'axe optique la face périphérique prévue éventuellement au raccordement mutuel des faces antérieure et postérieure de la zone d'ancrage de la lentille ; de préférence, ces dimensions sont limitées à un maximum de l'ordre de 50 µm ; les autres dimensions de la lentille peuvent être aisément déterminées par un Homme du métier, en fonction des dimensions de la cornée du globe receveur.

En rapport avec le mode d'incision préféré, indiqué plus haut, selon lequel on incise d'abord la cornée du globe receveur parallèlement à l'axe visuel de celui-ci avant de donner à l'incision une inclinaison propre à permettre l'insertion et la retenue de la zone d'ancrage de la lentille, la présente invention propose un kératotome destiné à la réalisation d'une incision annulaire dans une cornée.

On connaît déjà, par exemple du fait de US-A-4 423 728 qui sert de base à la subdivision de la revendication 21 en préambule et partie caractérisante, un kératotome comportant au moins une lame tournant autour d'un axe que l'on fait coïncider avec l'axe visuel du globe oculaire d'un patient et réglable en orientation pour réaliser une incision cylindrique ou tronconique dans la cornée. A chaque orientation de cette lame correspond cependant une distance différente de sa zone de sortie du kératotome par rapport à l'axe de rotation ou l'axe visuel du globe oculaire, si bien que ce kératotome est impropre à la réalisation de l'incision du type préféré précité, en vue de la pose de la lentille selon l'invention.

Le kératotome selon l'invention, comportant comme le kératotome décrit dans US-A-4 423 728 :
- un support extérieur tubulaire formant enveloppe, présentant un axe déterminé et comportant, pour son application sur une cornée, une partie de pied annulaire de révolution autour de l'axe du support et délimitant une surface géométrique de référence, définie comme la forme que présente la cornée lorsque le support est appliqué sur celle-ci par sa partie de pied,
- un corps intérieur tubulaire disposé coaxialement à l'intérieur du support, en retrait vers l'intérieur du support par rapport à la surface de référence,
- des moyens de guidage du corps à la rotation autour de l'axe du support par rapport au support, sur au moins 360°,
- des moyens d'entraînement du corps à la rotation autour de l'axe du support par rapport au support, sur au moins 360°,
- un couteau disposé à l'intérieur du corps, suivant une direction propre, et présentant dans un sens déterminé de sa direction propre une pointe coupante,
- des moyens de liaison entre le couteau et le corps, comportant eux-mêmes des moyens pour déplacer de façon réglée le couteau en translation suivant sa direction propre, par rapport au corps, entre une position de repos, dans laquelle il est placé en retrait vers l'intérieur du corps par rapport à la surface de référence et dans laquelle la pointe est tournée vers ladite surface de référence, et une position de coupe, dans laquelle la pointe forme une saillie à l'extérieur du corps par rapport à la surface de référence, de façon décalée par rapport à l'axe du support,
est caractérisé en ce que les moyens de liaison entre le couteau et le corps comportent en outre des moyens pour déplacer de façon réglée le couteau en orientation de sa direction propre par rapport au corps, entre plusieurs orientations déterminées dont une première orientation dans laquelle sa direction propre est parallèle à l'axe du support et une deuxième orientation dans laquelle sa direction propre est sécante de l'axe du support et oblique par rapport à celui-ci de telle sorte que, au moins en position de coupe, la pointe du couteau diverge dans ledit sens par rapport à l'axe, en conservant dans lesdites orientations un écartement sensiblement identique entre l'axe du support et une zone dans laquelle la pointe du couteau traverse la surface de référence en position de coupe.

Bien qu'un réglage de l'orientation du couteau entre un grand nombre d'orientations, par exemple de façon continue, ne sorte pas du cadre de la présente invention, lesdites orientations déterminées sont de préférence au nombre de deux, à raison desdites première et deuxième orientations, ce qui permet de réaliser le kératotome selon l'invention de façon particulièrement simple, c'est-à-dire à la fois de façon économique et de façon fiable, comme il apparaîtra par la suite.

De préférence, le kératotome selon l'invention comporte à l'intérieur de la partie de pied une paroi solidaire du corps et présentant vers l'extérieur du corps une face coïncidant avec la surface de référence, ladite paroi présentant des moyens de passage du couteau en position de coupe ; lors de la pénétration du couteau dans la cornée du globe receveur, cette paroi évite une déformation de cette cornée, par rapport à laquelle le kératotome est de préférence en outre retenu par le fait que la partie de pied délimite une cavité annulaire ouverte autour de la surface de référence, et par le fait que sont prévus des moyens pour mettre la cavité annulaire en dépression ; il en résulte à la fois l'impossibilité d'un déplacement relatif du kératotome et de la cornée à inciser et d'autre part une incision de cette dernière selon une géométrie parfaitement déterminée, ce qui assure d'une part une fermeture de l'incision sur elle-même lors de la rotation conjointe du corps et du couteau, en position de coupe, par rapport au support et d'autre part une parfaite coïncidence ultérieure de l'axe optique de la zone optique de la lentille avec l'axe visuel du globe receveur dès lors que le kératotome a été lui-même placé coaxialement par rapport à cet axe visuel préalablement à l'incision ; à cet effet, de préférence, ladite paroi comporte des moyens de vision à travers elle et/ou de repérage de positionnement par rapport à la cornée du globe receveur.

D'autres caractéristiques et avantages de la lentille pour épikératophakie et du kératotome selon l'invention ressortiront de la description ci-dessous, ainsi qu'une technique opératoire de mise en place de la lentille selon l'invention notamment par réalisation d'une incision au moyen du kératotome selon l'invention ; cette description est relative à des exemples non limitatifs de mise en oeuvre de l'invention, par ailleurs illustrés sur les dessins ci-annexés, qui font partie intégrante de la présente description.
- La figure 1 montre une vue d'une lentille selon l'invention en plan et de face, c'est-à-dire par les faces antérieures respectives de sa zone optique et de sa zone d'ancrage.
- La figure 2 illustre la mise en place d'une telle lentille sur la cornée d'un globe receveur, en une vue en coupe par un plan repéré en II-II, incluant l'axe optique de la zone optique de la lentille et l'axe visuel du globe receveur dans sa moitié de gauche, la figure 2 illustre l'incision réalisée à cet effet dans la cornée du globe receveur et la lentille avant l'insertion de la zone d'ancrage de cette dernière dans cette incision ; dans sa moitié de droite, la figure 2 montre la lentille et la cornée après insertion de la zone d'ancrage de la lentille dans l'incision de la cornée.
- La figure 3 montre, en une vue fortement agrandie, un détail de la lentille repéré en III à la figure 2.
- La figure 4 montre, en une vue analogue à celle de la figure 3, une variante de réalisation de la lentille illustrée aux figures 2 et 3.
- La figure 5 illustre, en une vue analogue à celle de la figure 2, la mise en place d'une lentille selon l'invention différente de celle qui est illustrée à la figure 2.
- La figure 6 montre, en une vue analogue à celle de la figure 3, un détail repéré en VI à la figure 5.
- La figure 7 montre, en une vue analogue à celle de la figure 4, une variante de réalisation de la lentille illustrée aux figures 5 et 6.
- La figure 8 montre un kératotome selon l'invention, en coupe par un plan repéré en VIII-VIII à la figure 9 et incluant l'axe de rotation relative du corps intérieur et du support extérieur, lequel axe coïncide avec l'axe visuel lors de l'utilisation du kératotome.
- La figure 9 montre un détail du kératotome, vu selon la flèche repérée en IX à la figure 8, parallèlement à l'axe de rotation relative du corps intérieur et du support extérieur.
- La figure 10 montre un autre détail du kératotome, en coupe par un plan repéré en X-X à la figure 8, lequel plan est perpendiculaire au plan repéré en VIII-VIII à la figure 9 et oblique par rapport à l'axe de rotation relative du corps intérieur et du support extérieur.
- La figure 11 montre une vue en coupe par un plan repéré en XI-XI à la figure 10, ce plan se confondant avec le plan repéré en VIII-VIII à la figure 9.
- La figure 12 montre le couteau du kératotome selon l'invention, en élévation dans un sens repéré par une flèche XII à la figure 11.

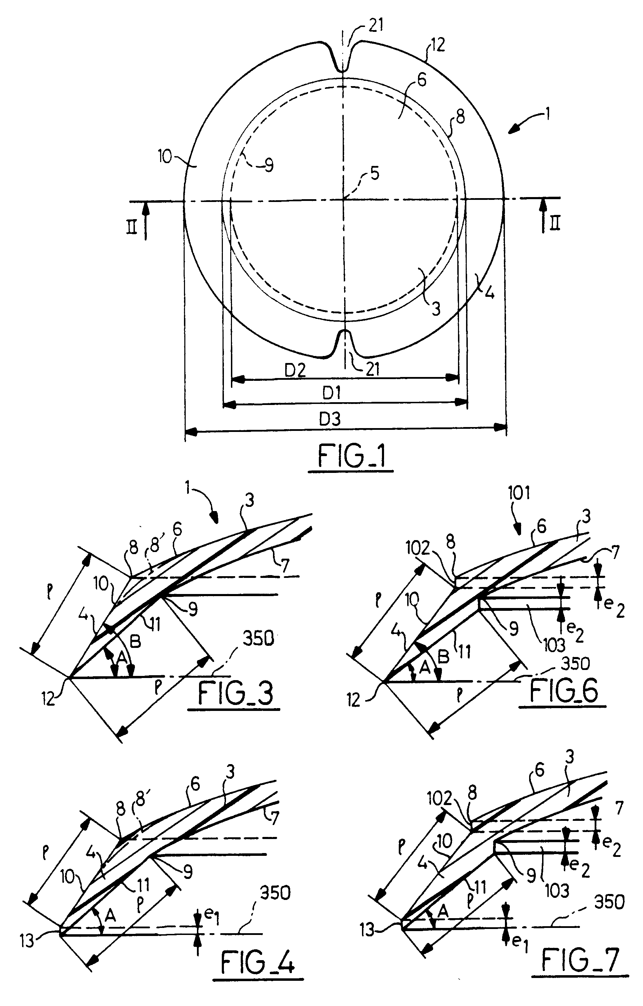

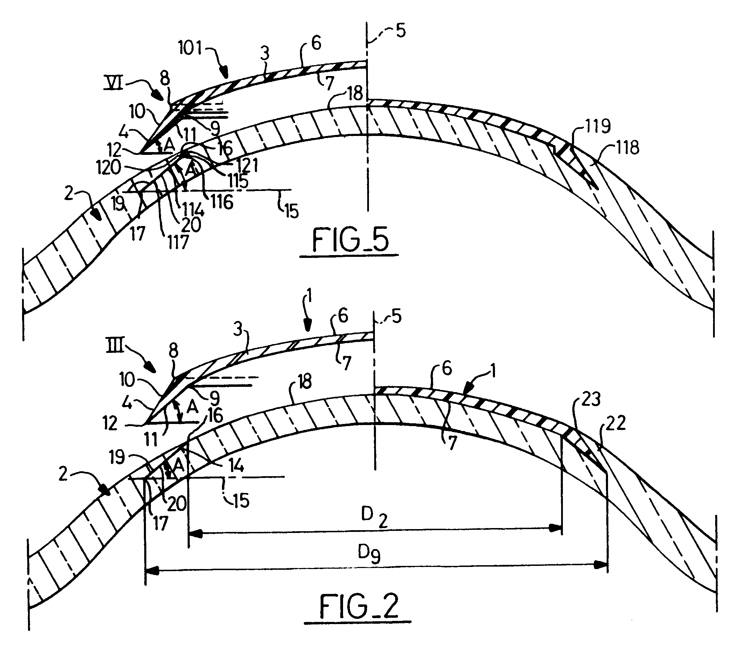

On se réfèrera en premier lieu aux figures 1 à 3, où l'on a désigné par 1 une lentille pour épikératophakie réalisée conformément à la présente invention, de préférence par moulage puis usinage, notamment au laser, d'au moins un polymère souple et bio-compatible choisi parmi les polymères naturels et les polymères de synthèse, comme par exemple du collagène ou un copolymère de collagène ; naturellement, cette lentille 1 doit être transparente et conserver sa transparence dans le temps et au contact avec la cornée d'un globe receveur, désigné par 2 à la figure 2.

De façon connue en elle-même, la lentille 1 comprend deux zones, à raison d'une zone optique 3 et d'une zone d'ancrage 4.

La zone optique 3 présente, de façon également connue, la forme d'une lentille présentant un axe optique 5 se confondant avec l'axe visuel du globe receveur lorsque la lentille est mise en place sur celui-ci. La zone optique 3 est délimitée par des faces respectivement antérieure 6, convexe et postérieure 7, concave qui, lorsque la lentille est mise en place sur la cornée du globe receveur, sont tournées respectivement vers l'extérieur de ce globe et vers la cornée 2 de celui-ci, dont la face postérieure 7 de la lentille 1 épouse intimement une zone de la surface 18. Les faces 6 et 7 présentent des géométries respectives déterminées, de façon connue par un Homme du métier, d'une part en fonction de cette possibilité d'appliquer intimement la face postérieure 7 contre la surface 18 de la cornée 2 du globe receveur et d'autre part en fonction de caractéristiques optiques prédéterminées, correspondant à la correction que l'on désire apporter au globe receveur ; dans le cas d'un globe receveur anastigmate, les deux faces 6 et 7 présentent la forme de calottes sphériques centrées l'une et l'autre sur l'axe optique 5 et on a précisément illustré un tel cas aux figures 1 à 3, où la zone optique 3 présente la forme d'une lentille divergente destinée à corriger la myopie, étant entendu que l'on ne sortirait pas du cadre de la présente invention en donnant à cette zone optique 3 la forme d'une lentille convergente pour corriger une hypermétropie ; dans le cas d'un globe receveur astigmate, l'une des deux faces de la zone optique 3, à savoir de préférence sa face postérieure 7, peut également être asphérique alors que son autre face, à savoir sa face antérieure 6, peut rester sphérique et centrée sur l'axe 5 mais on préfère dans un tel cas conformer la lentille selon l'invention comme il sera décrit en référence aux figures 5 à 7.

De préférence, l'épaisseur minimale de la zone optique 3 est au moins égale à 0,2 mm environ bien que l'on puisse admettre une épaisseur minimale plus faible, par exemple de l'ordre de 50 µm environ , cette épaisseur se mesurant suivant l'axe 5 dans le cas d'une zone optique 3 divergente ou en périphérie de la zone optique 3 dans le cas d'une zone optique 3 convergente, ce chiffre étant indiqué à titre d'exemple non limitatif.

Dans le sens d'un éloignement par rapport à l'axe 5, les deux faces 6 et 7 de la zone optique 3 sont délimitées par des périphéries respectives 8, 9 qui, dans le cas de faces 6 et 7 sphériques centrées sur l'axe 5, sont circulaires et situées dans des plans respectifs (non représentés) perpendiculaires à cet axe 5 et qui, dans le cas d'une face 6 ou 7 asphérique, présentent une forme différente d'une forme circulaire, bien que proche d'une telle forme, et déterminée par intersection de cette face 6 ou 7, respectivement, avec une face respectivement correspondante 10, 11, décrite ultérieurement, de la zone d'ancrage 4 ; naturellement, les diamètres respectifs des périphéries circulaires ou pratiquement circulaires (auquel cas on considère un diamètre moyen) 8 et 9 sont déterminés de telle façon que la zone optique 3 couvre l'intégralité du champ visuel du globe receveur ; pour un globe receveur d'humain adulte, la périphérie 8 de la face antérieure 6 présente un diamètre D₁ de l'ordre de 5 à 7 mm environ, ces chiffres étant indiqués à titre d'exemple non limitatif, alors que la périphérie 9 de la face postérieure 7 présente un diamètre D₂ inférieur d'un maximum de quelques dizièmes de millimètres à D₁, déterminé en fonction de la valeur de D₁ par des caractéristiques dimensionnelles de la zone d'ancrage 4 qui vont être décrites à présent.

Cette zone d'ancrage 4, caractéristique de la lentille selon l'invention, présente une forme générale annulaire de révolution autour de l'axe optique 5 et borde la zone optique 3 dans le sens d'un éloignement par rapport à cet axe optique 5.

Conformément à la présente invention, elle est disposée non seulement en saillie par rapport à la face postérieure 7 de la zone optique 3, mais également en retrait par rapport à la face antérieure 6 de celle-ci. Elle est ainsi délimitée, dans l'exemple illustré aux figures 1 à 3, par une face antérieure 10 et une face postérieure 11 l'une et l'autre tronconiques de révolution autour de l'axe 5 vers lequel elles se raccordent respectivement à la face antérieure 6 de la zone optique 3, directement le long de la périphérie généralement circulaire 8 de celle-ci, et à la face postérieure 7 de la zone optique 3, directement le long de la périphérie circulaire ou pratiquement circulaire 9 de celle-ci ; la périphérie 8, 9 de la face respectivement antérieure 6 ou postérieure 7 de la zone optique 3 présente ainsi la forme d'une arête respectivement saillante ou rentrante ; selon une variante illustrée en trait mixte à la figure 3, à propos du raccordement de la face antérieure 10 de la zone d'ancrage 4 avec la face antérieure 6 de la zone optique, cette arête pourrait être remplacée par une zone de transition telle que 8' de forme arrondie, respectivement convexe ou concave, se raccordant sans transition brusque aux faces correspondantes de la zone optique 3 et de la zone d'ancrage 4. A partir de leur raccordement respectivement à la face antérieure 6 de la zone optique 3 et à la face postérieure 7 de celle-ci, les deux faces 10 et 11 de la zone d'ancrage 4 convergent mutuellement dans le sens d'un éloignement par rapport à l'axe optique 5, et se raccordent mutuellement le long d'un bord périphérique 12 de la lentille 1, lequel bord périphérique 12 présente dans l'exemple illustré aux figures 1 à 3 la forme d'une arête circulaire centrée sur l'axe 5 et située dans un plan (non illustré) perpendiculaire à cet axe 5 ; compte tenu de la fragilité que peut présenter une telle arête 12, on peut également adopter une variante de réalisation de la lentille 1 illustrée aux figures 1 à 3 selon laquelle, comme il est illustré à la figure 4, les faces antérieure 10 et postérieure 11 de la zone d'ancrage 4 se raccordent mutuellement non pas directement, mais par l'intermédiaire d'une face périphérique 13 de révolution autour de l'axe 5, et par exemple cylindrique de révolution autour de cet axe ; parallèlement à l'axe 5, cette face 13 présente cependant une dimension e₁ aussi limitée que possible, et par exemple de l'ordre de 50 µm au maximum, ce chiffre étant indiqué à titre d'exemple non limitatif ; en référence à l'axe 5, l'arête 12 ou la face 13 qui se substitue à elle présente un diamètre D₃ supérieur à D₁, et par exemple de l'ordre de 7 mm à 9 mm pour les valeurs de D₁ indiquées plus haut, ces chiffres étant donnés qu'à titre d'exemple non limitatif.

De façon caractéristique de la présente invention, les faces antérieure 10 et postérieure 11 de la zone d'ancrage 4 présentent, lorsqu'elles sont vues en coupe par un demi-plan limité par l'axe optique 5 comme c'est le cas aux figures 2 et 3 ou, en variante, à la figure 4, une forme et des dimensions au moins approximativement identiques, à savoir dans l'exemple illustré, la forme de segments de droites de même longueur l, la valeur de l pouvant être avantageusement de l'ordre de 0,5 mm à 1,5 mm environ ; ces valeurs ne sont indiquées qu'à titre d'exemple non limitatif et correspondent d'une part aux valeurs de D₁ et D₃ indiquées plus haut, et d'autre part à des inclinaisons respectives A et B de la face postérieure 11 et de la face antérieure 10, par rapport à un plan 350 perpendiculaire à l'axe 5, comprises entre 40° et 45° environ et entre 50° et 55° environ, ces chiffres étant également indiqués à titre d'exemple non limitatif ; actuellement, on préfère pour A une valeur de 42°, en adoptant pour B une valeur qui en découle compte tenu des autres caractéristiques dimensionnelles de la lentille 1, la valeur de B étant naturellement supérieure à la valeur de A tout en restant sensiblement inférieure à 90°, mais on ne sortirait pas du cadre de la présente invention en adoptant d'autres valeurs ; de même, on ne sortirait pas du cadre de la présente invention en adoptant pour les faces 10 et 11 des formes différentes d'une forme tronconique de révolution autour de l'axe optique 5 dès lors que l'on conserverait la convergence mutuelle de ces faces dans le sens d'un éloignement par rapport à l'axe 5, avec raccordement direct, comme il est illustré aux figures 2 et 3, ou pratiquement direct, comme il est illustré à la figure 4, de ces faces dans le sens d'un éloignement par rapport à l'axe optique 5, et en conservant une identité au moins approximative des formes et dimensions de ces faces 10 et 11 lorsqu'elles sont vues en coupe par un demi-plan limité par l'axe optique 5 ; dans un tel cas, les dimensions 1 doivent être considérées comme la longueur du développement rectiligne des sections des faces 10 et 11 ainsi conçues par un demi-plan incluant l'axe 5 et A et B sont des inclinaisons moyennes des faces 10 et 11 par rapport à un plan perpendiculaire à l'axe 5 ; lorsque la face respectivement antérieure 10 ou postérieure 11, par ailleurs tronconique, de la zone d'ancrage 4 se raccorde à la face respectivement antérieure 6 ou postérieure 7 de la zone optique 3 par une zone de transition arrondie telle que 8′, le développement de cette zone de transition telle que 8′ lorsqu'elle est vue en coupe par un demi-plan limité par l'axe optique 5 entre environ pour moitié dans le calcul de la valeur de l, dont ce développement peut représenter jusqu'à la moitié, et environ pour moitié dans le calcul de D₁, en regard duquel ce développement est en fait pratiquement négligeable.

Dans ces conditions, la lentille 1 décrite en référence aux figures 1 à 3, ou sa variante décrite en référence à la figure 4, peut être mise en place sur la cornée 2 d'un globe receveur, par un procédé qui va être décrit à présent en référence à la figure 2.

Dans une première phase de ce procédé, après avoir retiré l'epithélium par exemple au moyen d'alcool à 90° dans la zone de la surface 18 de la cornée 2 destinée à recevoir la lentille 1, on réalise dans la cornée 2 une incision 14, illustrée dans la partie de gauche de la figure 2, présentant une forme et des dimensions correspondant à celles de la face postérieure 11 de la zone d'ancrage 4 de la lentille 1 ; en d'autres termes, si l'on se réfère spécifiquement aux exemples décrits dans lesquels la face postérieure 11 présente une forme tronconique de révolution autour de l'axe 5, entre un cercle de diamètre D₂ (périphérie 9 de la face postérieure 7 de la zone optique 3) et un cercle de diamètre D₃ (diamètre de l'arête périphérique 12 ou de la face périphérique 13), avec une inclinaison A par rapport à un plan perpendiculaire à l'axe optique 5, on réalise une incision 14 tronconique de révolution autour de l'axe visuel du globe optique 2 (cet axe se confondant avec l'axe optique 5 à la figure 2), avec une inclinaison égale à A vis-à-vis d'un plan 15 perpendiculaire à l'axe visuel, et cette incision 14 présente à son débouché à l'extérieur de la cornée 2 la forme d'un cercle 16 centré sur l'axe 5 et situé dans un plan (non référencé) perpendiculaire à cet axe 5, avec un diamètre égal à D₂, alors qu'elle se termine à l'intérieur de la cornée 2 par une arête de fond circulaire 17 également centrée sur l'axe 5 et située dans un plan (plan 15 dans l'exemple illustré) perpendiculaire à cet axe 5, avec un diamètre D₉ au moins égal à D₃, et de préférence supérieur à D₃ par exemple d'au moins 0,5mm environ ; on remarque que, compte tenu des dimensions préférées indiquées plus haut et dans le cas d'un globe oculaire d'humain adulte, l'incision 14 ainsi réalisée, bien qu'elle débouche dans la surface 18 de la cornée 2 dans une zone encore centrale de celle-ci, d'épaisseur comparativement réduite, s'enfonce dans la cornée 2 obliquement par rapport à la surface 18 de celle-ci, vers des zones périphériques d'épaisseur comparativement plus importante si bien qu'elle n'affaiblit mécaniquement que peu la cornée 2.

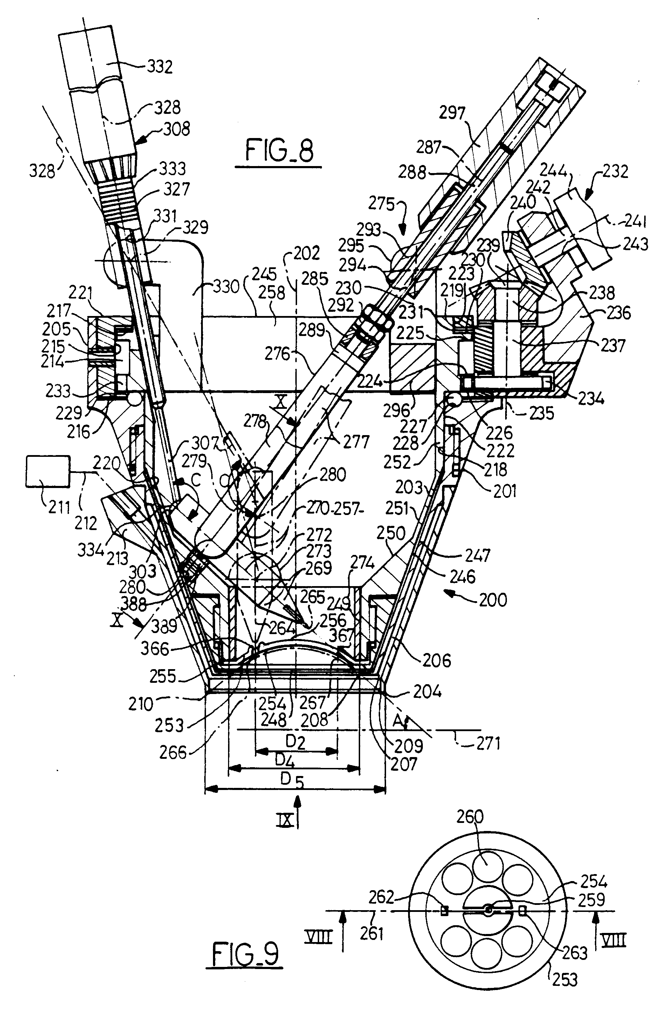

L'incision 14 est ainsi réalisée de façon continue tout autour de la cornée 2, par exemple au moyen du kératotome qui sera décrit plus loin en référence aux figures 8 à 12, et présente ainsi deux flancs 19, 20 l'un et l'autre tronconiques de révolution autour de l'axe 5 et initialement identiques.

Après avoir ainsi réalisé l'incision 14, on présente la lentille 1 en regard de la cornée 2 et, par des mouvements relatifs imprimés en poussant la lentille 1 au moyen d'un tampon, on insère la zone d'ancrage 4 à l'intérieur de l'incision 14, entre les flancs 19 et 20 de celle-ci ; pour faciliter cette insertion, de préférence, on prévoit dans la zone d'ancrage 4, par exemple de façon diamétralement opposée en référence à l'axe optique 5 de la zone optique 3, des encoches 21 débouchant dans l'arête périphérique 12 (ou dans la face périphérique 13, suivant le cas) ainsi que dans les faces antérieure 10 et postérieure 11 de la zone d'ancrage 4, étant entendu que l'on pourrait également se dispenser de telles encoches 21 ou en prévoir un nombre différent de deux ; on remarquera toutefois que, dans le cas d'une lentille destinée à corriger un astigmatisme, deux encoches diamétralement opposées en référence à l'axe optique 5, comme il est illustré, constituent un moyen commode de repérage de positionnement angulaire de la lentille autour de l'axe optique 5, étant entendu que d'autres moyens pourraient leur être substitués à cet effet.

Au cours de cette insertion, la zone d'ancrage 4 provoque un écartement mutuel progressif des deux flancs 19 et 20 de l'incision 14 et, à la fin de cette insertion, alors que l'axe optique 5 de la zone optique 3 coïncide avec l'axe visuel de l'oeil et que la face postérieure 7 de la zone optique 3 épouse la zone de la surface 18 de la cornée 2 située à l'intérieur du cercle 16, la face postérieure 11 de la zone d'ancrage 4 épouse le flanc 19 de l'entaille 14, sans déformation de la cornée à ce niveau, l'arête périphérique 12 (ou la face périphérique 13, assimilable avec une bonne approximation à une telle arête) épouse pratiquement le cercle 17 de raccordement mutuel des deux flancs 19 et 20 de l'incision 14 alors que le flanc 20 de cette incision 14 épouse la face antérieure 10 de la zone d'ancrage 4, ce qui déforme légèrement vers l'extérieur une lame 22 de la cornée 2, située entre le flanc 20 de l'entaille 14 et la surface 18 de la cornée 2, comme le montre la figure 2 dans sa partie de droite ; des études ont démontré que la déformation ainsi subie par la cornée 2 était étroitement limitée à des zones voisines du cercle 17, et parfaitement tolérable ; en particulier, vers l'axe 5, la lame 22 se termine par une arête circulaire 23, issue de l'écartement de cette lame 22 au niveau du cercle 16, à la périphérie généralement circulaire 8 de la face antérieure 6 de la zone optique 3, à laquelle la lame 22 se raccorde ainsi sans former de surépaisseur génératrice de gêne ultérieure au contact des paupières, et pratiquement sans discontinuité susceptible d'être comblée ultérieurement par du collagène généré par la cornée 2 au prix de la formation d'un anneau blanchâtre ou de donner lieu à la formation ultérieure d'un bourrelet d'épithélium, générateur de gêne.

Après que l'on ait ainsi effectué l'insertion de la zone d'ancrage 4 de la lentille 1 dans l'incision 14, on effectue à ce niveau une suture dans des conditions habituelles et en prenant les précautions habituelles, puis on met en place un pansement ensuite conservé pendant le temps nécessaire à une épithélisation de la face antérieure 6 de la lentille 1, en pratique quelques jours ; il est également possible de se dispenser de toute suture, compte tenu d'un effet d'encastrement résultant de la bonne adaptation mutuelle des formes respectives de la zone d'ancrage 4 de la lentille et des zones correspondantes de la cornée 2, à savoir notamment de la lame 22 de celle-ci.

Pour des raisons de commodité d'incision, quelle que soit la forme des faces antérieure et postérieure de la zone optique de la lentille selon l'invention, et pour des raisons de facilité de conception et de réalisation de celle-ci lorsque l'une au moins de ces faces est asphérique, on préfère cependant utiliser une lentille selon l'invention d'un type illustré aux figures 5 et 6 ou, en variante, à la figure 7.

La lentille selon l'invention 101 illustrée aux figures 5 et 6, ou sa variante illustrée à la figure 7, présente de grandes analogies avec la lentille illustrée aux figures 1 à 3 ou sa variante illustrée à la figure 4, si bien que l'on retrouve aux figures 5 et 6 les références 6 à 12 et à la figure 7 la référence 13, pour désigner des parties de la lentille 101 ou de sa variante identiques aux parties qui ont été décrites sous les mêmes références, à propos de la lentille 1 ou de sa variante illustrée à la figure 4 ; en particulier, les dispositions géométriques caractéristiques de l'invention ainsi que les caractéristiques dimensionnelles indiquées à titre d'exemple non limitatif, à propos du mode de réalisation des figures 1 à 3 et de la variante illustrée à la figure 4, peuvent être reprises à propos de la lentille 101 illustrée aux figures 5 et 6 et de sa variante illustrée à la figure 7.

La lentille 101 illustrée aux figures 5 et 6 et sa variante illustrée à la figure 7 ne diffèrent en fait de la lentille 1 illustrée aux figures 1 à 3 et de sa variante illustrée à la figure 4, respectivement, que par le mode de raccordement des faces antérieure 10 et postérieure 11 de la zone d'ancrage 4 aux faces antérieure 6 et postérieure 7, respectivement, de la zone optique 3.

Dans le cas de la lentille 101 illustrée aux figures 5 et 6 comme dans le cas de sa variante illustrée à la figure 7, la face antérieure de la zone d'ancrage 4 se raccorde à la périphérie 8 de la face antérieure 6 de la zone optique 3 par une face 102 annulaire de révolution autour de l'axe optique 5 et par exemple cylindrique de révolution autour de cet axe, laquelle face 102 est tournée dans le sens d'un éloignement par rapport à l'axe 5 de façon à former un décrochement par rapport à la face antérieure 6 de la zone optique 3 ; parallèlement à l'axe 5, cette face 102 présente une dimension e₂ faible par rapport aux autres dimensions de la lentille 101, c'est-à-dire du même ordre de grandeur que la dimension e₁ décrite en référence à la figure 4 et que l'on retrouve à la figure 7, à savoir une valeur de l'ordre de 50 µm environ au maximum, cette valeur étant indiquée à titre d'exemple non limitatif. Quant à elle, la face postérieure 11 de la zone d'ancrage 4 se raccorde à la périphérie 9 de la face postérieure 7 de la zone optique 3 par une face 103 également de révolution autour de l'axe 5 et par exemple cylindrique de révolution autour de cet axe, vers lequel cette face 103 est toutefois tournée de façon à former une saillie par rapport à la face postérieure 7 de la zone optique 3 ; parallèlement à l'axe 5, la face 103 présente sensiblement la même dimension e₂ que la face 102. Lorsque les faces antérieure 6 et postérieure 7 de la zone optique 3 sont sphériques, leurs périphéries 8, 9 sont circulaires mais lorsque l'une, au moins, d'entre elles est asphérique, sa périphérie 8, 9 présente une forme différente d'une forme circulaire bien que proche d'une telle forme, en se situant toutefois sur un cylindre de révolution autour de l'axe 5 lorsque, respectivement, la face 102 ou la face 103 est elle-même cylindrique de révolution autour de cet axe ; naturellement, les faces 102 et 103 présentent les mêmes diamètres D₁, D₂ que les périphéries 8 et 9 des faces antérieure et postérieure 6, 7 de la zone optique 3, respectivement, lorsque ces faces 102 et 103 sont l'une et l'autre cylindriques de révolution autour de l'axe 5, comme il est préféré ; dans le cas d'une forme différente d'une forme cylindrique bien que restant de révolution autour de l'axe 5, chacune des faces 102 et 103 va en s'élargissant à partir de son raccordement avec la périphérie 8, 9 de la face antérieure 6 ou postérieure 7 de la zone optique 3, respectivement, jusqu'à se raccorder respectivement à la face antérieure 10 de la zone d'ancrage 4 ou à la face postérieure 11 de cette zone d'ancrage 4.

On remarquera qu'une forme cylindrique, de révolution, des deux faces 102 et 103 permet d'assurer de façon géométriquement simple le raccordement d'une zone d'ancrage 4 présentant une forme rigoureusement de révolution autour de l'axe 5, notamment par ses faces antérieure et postérieure 11, avec une zone optique 3 présentant une forme différente d'une forme rigoureusement de révolution autour de cet axe 5, comme c'est le cas lorsque l'une, au moins, de ces faces 6 et 7 présente une asphéricité ; en celà, la lentille 101 illustrée aux figures 5 et 6, ou sa variante illustrée à la figure 7, convient mieux que la lentille illustrée aux figures 1 à 3, ou sa variante illustrée à la figure 4, à la correction des astigmatismes ; on remarquera également que dans un tel cas, les encoches 21 que l'on peut avantageusement retrouver sur la lentille 101 illustrée aux figures 5 et 6 ou sur sa variante illustrée à la figure 7 peuvent servir pour repérer l'axe d'astigmatisme, et orienter de façon appropriée la lentille lors de sa mise en place sur la cornée 2 ; naturellement, la face 102 ou 103 assurant la liaison entre la zone d'ancrage 4 et la face ainsi asphérique 6 ou 7 de la zone optique 3 présente une dimension e₂ variable entre un maximum et un minimum mais, dans toute la mesure du possible, on conserve des valeurs aussi voisines que possible pour les dimensions que présentent respectivement les faces 102 et 103 parallèlement à l'axe 5.

Quelles que soient les formes adoptées pour les faces 102 et 103, on coordonne ces formes de telle sorte que, dans une vue en coupe par un demi-plan incluant l'axe 5, les faces 102 et 103 occupent des positions aussi voisines que possible respectivement par rapport à la face antérieure de la zone d'ancrage 4 et par rapport à la face postérieure 11 de celle-ci.

Dans ces conditions, la mise en place de la lentille 101 illustrée aux figures 5 et 6 ou de sa variante illustrée à la figure 7 s'effectue de la façon illustrée à la figure 5, c'est-à-dire par aménagement préalable, dans la cornée 2, par la face 18 de celle-ci et après retrait localisé de l'épithélium, d'une incision 114 réalisée, comme l'incision 14, sans enlèvement de matière et présentant une forme correspondant à celle de l'ensemble de la face postérieure 11 de la zone d'ancrage 4 et de la face 103 assurant son raccordement à la périphérie 9 de la face postérieure 7 de la zone optique 3 de la lentille, comme il ressort de l'examen de la figure 5 dans sa partie de gauche.

Plus précisément, l'incision 114 débouche dans la face 18 de la cornée 2 par un cercle 16 identique à celui qui a été décrit en référence à la figure 2, c'est-à-dire présentant le diamètre D₂ de la périphérie circulaire ou approximativement circulaire 9 de la face postérieure 7 de la zone optique 3 ; vers l'interieur de la cornée 2 à partir de ce cercle 16, et lorsqu'elle est vue en coupe par un demi-plan limité par l'axe 5, l'incision 14 reproduit la forme de la face 103 et de la face postérieure 11 de la zone d'ancrage 4 ; en d'autres termes, lorsque la face 103 présente comme il est préféré une forme cylindrique de révolution autour de l'axe 5, l'incision 114 présente de façon directement adjacente au cercle 16 par lequel elle débouche dans la face 18 de la cornée 2 une zone 115 cylindrique de révolution autour de l'axe 5, d'un diamètre égal à D₂, et délimitée respectivement vers l'axe 5 et dans le sens d'un éloignement par rapport à celui-ci par des flancs 120 et 121 l'un et l'autre cylindriques de révolution autour de cet axe 5 avec, parallèlement à cet axe, une dimension égale à la dimension e₂ ou à une valeur moyenne de la dimension e₂ lorsque la face postérieure 7 de la zone optique 3 de la lentille est asphérique ; vers l'intérieur de la cornée 2, cette zone 115 se raccorde par une arête circulaire 116, identique au cercle 16, à une zone 117 reproduisant à l'identique l'incision 14 décrite en référence à la figure 2, c'est-à-dire délimitée par deux flancs 19 et 20 tronconiques de révolution autour de l'axe 5 et reproduisant la face postérieure 11 de la zone d'ancrage 4, à l'identique si ce n'est qu'ils se prolongent plus loin que celle-ci de l'axe 5, lesquels flancs 19 et 20 se raccordent mutuellement par une arête circulaire de fond 17 dans le sens d'un éloignement par rapport à l'axe optique 5.

L'insertion de la lentille 101 illustrée aux figures 5 et 6, ou de sa variante illustrée à la figure 7, dans l'incision 114 s'effectue comme on l'a dit plus haut à propos de la lentille 1 illustrée aux figures 1 à 3, ou de sa variante illustrée à la figure 4, et de l'incision 14. Préalablement à cette insertion, l'incision 114 se présente à l'état fermé comme il est illustre dans la partie de gauche de la figure 5, et on y insère progressivement la zone d'ancrage 4, par des mouvements relatifs de la lentille et de la cornée 2, en écartant mutuellement d'abord les flancs 120 et 121 puis les flancs 19 et 20 jusqu'à ce que la lentille 101, ou sa variante illustrée à la figure 7, parvienne dans la position illustrée dans la partie de droite de la figure 5, dans laquelle la face postérieure 7 de la zone optique 3, la face 103, la face postérieure 11 de la zone d'ancrage 4, l'arête périphérique 12 ou la face périphérique 13, la face antérieure 10 de la zone d'ancrage 4, et la face 102 épousent respectivement la zone de la surface 18 de la cornée 2 située à l'intérieur du cercle 16, le flanc 120 de la zone 115 de l'incision 14, le flanc 19 de la zone 117 de celle-ci, l'arête de fond 17 avec un léger jeu, le flanc 20 de la zone 117 de l'incision 114, et le flanc 121 de la zone 115 de celle-ci en écartant une lamelle 118 de cornée 2 située entre l'incision 114 et la surface 18 de la cornée ; pour des raisons géométriques aisément compréhensibles, cette lame 118 se raccorde par une arête circulaire 119, correspondant au raccordement du flanc 117 de la zone 115 de l'incision 114 avec la surface 18 de la cornée 2 au niveau du cercle 16 lors de la réalisation de l'incision 14, à la périphérie 8 de la face antérieure 6 de la lentille pratiquement sans discontinuité, c'est-à-dire avec les avantages déjà décrits en référence à la figure 2.

Ensuite, la zone d'ancrage 4 est suturée avec la cornée 2 de façon connue, avec les précautions habituelles, puis un pansement est posé pour le temps nécessaire à l'épithélisation de la face antérieure 6 de la lentille 1 ; la suture de la zone d'ancrage 4 sur la cornée 2 n'est cependant pas indispensable, la zone d'ancrage 4 étant retenue sous la lame 22 de cornée par un effet d'encastrement.

Naturellement, les modes de réalisation d'une lentille selon l'invention qui viennent d'être décrits ne constituent que des exemples non limitatifs, par rapport auxquels on pourra prévoir de nombreuses variantes ne sortant pas du cadre de la présente invention ; en particulier, des variantes pourraient porter sur le mode de raccordement des faces antérieure et postérieure de la zone d'ancrage avec les faces antérieure et postérieure de la zone optique, respectivement, l'un de ces raccordements pouvant être direct comme on l'a décrit en référence aux figures 1 à 4 et l'autre être indirect comme on l'a décrit en référence aux figures 5 à 7.

Quel que soit le mode de réalisation adopté pour la lentille selon l'invention, la forme de l'incision à réaliser pour la recevoir dans la cornée 2 peut être aisément déterminée par un Homme du métier.

Cette incision peut être réalisée à la main, ce qui nécessite une grande adresse, mais aucun des kératotomes ou trépans cornéens actuellement connus ne permet de la réaliser.

La présente invention propose par conséquent un kératotome propre à réaliser une telle incision, et un exemple non limitatif de réalisation d'un tel kératotome va être décrit à présent en référence aux figures 8 à 12, et en premier lieu à la figure 8 où l'on a désigné par 200 ce kératotome.

En vue de sa préhension manuelle par le chirurgien et de son positionnement sur la surface 18 de la cornée 2 à inciser, ce kératotome 200 comporte un support extérieur 201 présentant la forme générale d'une paroi tubulaire de révolution autour d'un axe 102 et enveloppant un corps intérieur 203 présentant également la forme d'une paroi tubulaire de révolution autour de l'axe 202, avec possibilité de rotation relative autour de cet axe 202 à l'exclusion de toute autre possibilité de déplacement relatif.

Plus précisément, le support extérieur 201 présente une forme générale tronconique de révolution autour de l'axe 202, et diverge d'une partie de pied 204, annulaire de révolution autour de l'axe 202 et destinée à s'appliquer sur la surface 18 de la cornée 2 à inciser, autour de l'emplacement de l'incision 14 ou 114 à réaliser, jusqu'à une partie de tête 205 également annulaire, de révolution autour de l'axe 202 et destinée quant à elle à la préhension manuelle du support extérieur 201, par la main non dominante du chirurgien.

Pour permettre une immobilisation relative du support extérieur 201 et de la cornée 2 à inciser, la paroi constituant le support extérieur 201 est dédoublée dans une zone adjacente à la partie de pied 204 de façon à délimiter à proximité immédiate de cette partie de pied 204 une cavité 206 annulaire, de révolution autour de l'axe 202 et s'ouvrant dans la partie de pied 204 par une fente annulaire 207, de révolution autour de l'axe 202. Cette fente 207 est délimitée, respectivement dans le sens d'un rapprochement vis-à-vis de l'axe 202 et dans le sens d'un éloignement par rapport à celui-ci, par deux portées 208, 209 annulaires de révolution autour de l'axe 202 et se situant sur une même surface de référence 210 reproduisant la forme de la surface 18 de la cornée 2, supposée dans une relation de coïncidence de l'axe visuel avec l'axe 202 ; naturellement, compte tenu des différences de forme qui peuvent exister entre les surfaces 18 de différentes cornées 2, la surface de référence 210 est choisie de façon à correspondre à la majorité des cas, et de telle sorte que les surfaces 18 de cornées 2 présentant des formes légèrement différentes puissent s'y adapter grâce à un effet d'aspiration vers l'intérieur de la cavité 206, par la fente 207, résultant du raccordement de cette cavité 206 à des moyens 211 permettant d'y établir une dépression relative, lesquels moyens 211 sont disjoints du kératotome 200 et raccordés à la cavité 206 par un embout de raccordement 213 de celle-ci, placé en saillie sur le support extérieur 201, dans le sens d'un éloignement par rapport à l'axe 202, entre les parties de pied 204 et de tête 205, et par un conduit souple 212. A titre d'exemple non limitatif, les portées 208 et 209 peuvent être délimitées, respectivement dans le sens d'un rapprochement vis-a-vis de l'axe 202 et dans le sens d'un éloignement par rapport à celui-ci, par un cercle (non référencé) de diamètre D₄ supérieur à D₂ et de préférence à D₃, et par exemple de l'ordre de 11,4 mm, et par un cercle (non référencé) de diamètre D₅ supérieur à D₄ et par exemple de l'ordre de 15,5 mm.

Par sa partie de tête 205, le support extérieur 201 délimite une gorge 214 annulaire de révolution autour de l'axe 202 et ouverte vers ce dernier. Plus précisément, la gorge 214 est délimitée dans le sens d'un éloignement par rapport à l'axe 202 par une face de fond 215 cylindrique de révolution autour de cet axe avec un diamètre aussi grand que possible compte tenu des dimensions de la partie de tête 205 ; vers la partie de pied 204 et à l'opposé de celle-ci, respectivement, la gorge 214 est délimitée par des faces annulaires planes 216, 217, de révolution autour de l'axe 202 auquel ces faces 216 et 217 sont perpendiculaires ; ces faces 216 et 217 raccordent la face de fond 215 de la gorge 214 à des faces respectives 218, 219 du support extérieur 201, lesquelles sont cylindriques de révolution autour de l'axe 202 vers lequel elles sont tournées ; la face 218 raccorde elle-même la face 216 à une face 220 du support 201, laquelle est tronconique de révolution autour de l'axe 202 et raccorde, en convergeant, la face 218 à la portée 208 de la partie de pied 204 du support 201 ; la face 219 raccorde quant à elle la face 217 à une face 221 annulaire, plane, de révolution autour de l'axe 202, laquelle face 221 est tournée à l'opposé de la partie de pied 204 et délimite le support extérieur 201 à l'opposé de cette partie de pied 204.

Le support 201 qui vient d'être décrit est avantageusement constitué de plusieurs pièces assemblées mutuellement par des moyens permettant de les dissocier à volonté, et par exemple par vissage, de telle sorte qu'on puisse le nettoyer aisément et, le cas échéant, changer sa partie de pied 204 contre une partie de pied dont les portées 208 et 209 sont conformées différemment, par exemple pour permettre l'utilisation d'un même kératotome 200 sur des globes occulaires présentant des conformations géométriques très différentes.

Le support extérieur 201 coopère par la gorge 214 et la face 218 avec le corps intérieur 203 pour assurer le guidage de ce dernier à la rotation autour de l'axe 202 sans autre possibilité de mouvement relatif.

A cet effet, le corps 203 est délimité, dans le sens d'un éloignement par rapport à l'axe 202, notamment par une face 222 cylindrique de révolution autour de l'axe 202 avec un diamètre sensiblement identique à celui de la face 218, avec laquelle cette face 202 est placée en contact intime mais avec possibilité de glissement de telle sorte que ce contact mutuel assure le guidage du corps 203 à la rotation autour de l'axe 202, et par une face 223 également cylindrique de révolution autour de l'axe 2 et présentant quant à elle un diamètre légèrement inférieur à celui de la face 219 en regard de laquelle elle est placée, sans contact mutuel ; entre les faces 218 et 219, le corps 203 présente en saillie vers l'extérieur par rapport à l'axe 202 deux épaulements 224, 225 de forme générale annulaire de révolution autour de l'axe 202 et s'engageant dans la gorge 214, respectivement à proximité de la face 216 de celle-ci et à proximité de la face 217 de celle-ci, mais sans contact avec l'une quelconque des faces 215 à 217 de la gorge 214 ; cependant, l'épaulement 224 le plus proche de la face 216 de la gorge 214 présente vers cette face 216 une face 226 annulaire, plane, de révolution autour de l'axe 202 auquel elle est perpendiculaire, et le corps intérieur 203 s'appuie sur le support extérieur 201, dans un sens de l'axe 202 allant de la face 226 vers la face 216, par contact de la face 226 avec un jeu de billes 227 elles-même engagées partiellement dans une gorge 228 creusée dans la face 216 et présentant une forme annulaire de révolution autour de l'axe 202 ; les billes 227 sont avantageusement retenues, dans une relation d'écartement angulaire mutuel constant, en référence à l'axe 202, par une cage 229 en matériau à faible coefficient de frottement, interposée entre les faces 226 et 216 ; l'épaulement 225 le plus proche de la face 217 présente quant à lui vers celle-ci une face 230 annulaire de révolution autour de l'axe 202 et plane, perpendiculaire à cet axe 202, et entre les faces 217 et 230 sont intercalées des rondelles 231 annulaires, de révolution autour de l'axe 202, réalisées en un matériau à faible coefficient de frottement et permettant de retenir les deux épaulements 224 et 225 sans jeu, parallèlement à l'axe 202, entre les faces 216 et 217 du support extérieur 201.

Le corps 203 est ainsi susceptible de tourner autour de l'axe 202, à l'intérieur du support 201, sur au moins 360° et, de préférence, sans limitation. Naturellement, d'autres moyens pourraient être choisis pour assurer le guidage du corps 203 et du support 201 à la rotation relative autour de l'axe 202, de même que le corps 203 et le support 201 pourraient présenter des formes différentes des formes décrites et illustrées.

Pour permettre au chirurgien de provoquer à volonté une telle rotation sur au moins 360° sont prévus des moyens d'entraînement 232 présentant une conception en elle-même connue, par exemple la conception décrite dans le brevet européen accordé N° 0 047 190, étant entendu que d'autres moyens pourraient également être prévus à cet effet.

Pour coopérer avec ces moyens d'entraînement 232, l'épaulement 224 présente dans le sens d'un éloignement par rapport à l'axe 202, à l'intérieur de la gorge 214, la forme d'une couronne dentée 233 qui s'engrène avec un pignon 234 monté à la rotation, par rapport au support extérieur 201, autour d'un axe 235 parallèle à l'axe 202 et décalé par rapport à la gorge 214 dans le sens d'un éloignement par rapport à l'axe 202 ; à cet effet, la face de fond 215 est creusée entre les axes 202 et 235 d'une encoche (non référencée) de passage du pignon 234 et la partie de tête 205 du support 201 porte de façon solidaire, en saillie dans le sens d'un éloignement par rapport à l'axe 202, en regard de cette encoche, une chaise double 236 qui définit un premier palier 237 situé entre le pignon 234 et la face 221 du support 201 et recevant à la rotation relative autour de l'axe 235, sans autre possibilité de déplacement relatif, un arbre 238 solidaire du pignon 234 ; à l'opposé du pignon 234 par rapport au palier 237, l'arbre 238 porte de façon solidaire un pignon d'angle 239 qui lui-même engrene avec un autre pignon d'angle 240 situé à l'opposé de l'axe 202 par rapport à l'axe 235 et présentant un axe 241 oblique par rapport à l'axe 235 ; pour assurer un guidage du pignon 240 à la rotation autour de son axe 241, par l'intermédiaire d'un arbre 242 que le pignon 240 porte coaxialement de façon solidaire, la chaise double 236 définit un deuxième palier 243 d'axe 241, que l'arbre 242 traverse de part en part pour présenter, à l'opposé de ce palier 243 par rapport au pignon 240, un bouton de manoeuvre manuelle 244 ou des moyens de liaison avec un moteur électrique ou pneumatique d'entraînement (non représenté).

De façon non représentée mais aisément concevable par un Homme du métier, des moyens d'indexage sont prévus pour, au moins, repérer la position angulaire du corps intérieur 203 autour de l'axe 202 par rapport au support extérieur 201, par exemple sous la forme de graduations portées par la face 221 du support 201 et par une face annulaire plane 245, de révolution autour de l'axe 202, à laquelle la face 223 du corps 203 se raccorde à l'opposé de son raccordement avec l'épaulement 225 et qui est placée coplanairement avec la face 221, dans la même orientation que celle-ci ; de préférence, cependant, on prévoit en outre des moyens pour immobiliser à volonté le corps 203 à la rotation autour de l'axe 202 par rapport au support 201 dans une position déterminée, par exemple par verrouillage provisoire du bouton d'entraînement manuel 244 comme il est décrit dans le brevet européen délivré précité.

A l'opposé de son raccordement avec l'épaulement 224, la face 222 du corps 203 se raccorde à une face 246 de celui-ci, laquelle présente une forme tronconique de révolution autour de l'axe 202, est tournée dans le sens d'un éloignement par rapport à celui-ci, et se situe en regard de la face 220 du support extérieur 201, parallèlement à celle-ci et moyennant respect d'un jeu relatif continu 247 de façon à éviter tout contact mutuel entre les faces 246 et 220 et par conséquent toute entrave à la rotation relative du corps 203 et du support 201.

La face 246 s'étend ainsi de son raccordement à la face 222 jusqu'à son raccordement à une face annulaire plane 248, de révolution autour de l'axe 202 auquel cette face 248 est perpendiculaire, laquelle face 248 constitue la limite extrême du corps 203 vers la partie de pied 204 du support 201. Comme l'ensemble du corps 203, cette face extrême 248 est placée en retrait, vers l'intérieur du support 201, par rapport à la surface de référence 210 définie précédemment.

Vers l'axe 202, la face 248 du corps 203 raccorde la face 246 de celui-ci à une face 249 cylindrique de révolution autour de l'axe 202 vers lequel cette face 249 est tournée ; la face 249 raccorde la face 248 à une face 250 quant à elle de forme tronconique de révolution autour de l'axe 202 vers lequel elle est tournée, cette face 250 divergeant de son raccordement avec la face 249 à son raccordement avec une face 251 également tronconique de révolution autour de l'axe 202 vers lequel cette face 251 est tournée, laquelle face 251 présente une obliquité moindre que l'obliquité de la face 250 par rapport à l'axe 202 et en pratique identique à celle de la face 246 si bien que les faces 251 et 246 sont mutuellement parallèles ; la face 251 raccorde elle-même la face 250 à une face 252 quant à elle cylindrique de révolution autour de l'axe 202 vers lequel cette face 252 est tournée ; la face 252, parallèle à la face 218, raccorde ainsi la face 251 à la face 245 du corps 203. Le corps 203 délimite ainsi autour de l'axe 202 une cavité intérieure 257.

Comme le support 201, le corps 203 est avantageusement réalisé par assemblage mutuel de plusieurs pièces, avec possibilité de démontage en vue des opérations de maintenance ou de changement de caractéristiques dimensionnelles, liées notamment à la volonté d'utiliser le même kératotome 200 sur des globes présentant des dimensions très différentes.

En effet, comme le support 201 dans sa partie de pied 204, le corps 203 est propre à épouser la surface 18 de la cornée 2 à inciser, par une paroi 253 présentant la forme générale d'une calotte de révolution autour de l'axe 202, laquelle paroi 253 ferme le corps 203 vers la partie de pied 204 du support 201, à l'intérieur de la face 249 et présente à cet effet vers la partie de pied 204 une face 254 épousant la surface de référence 210, c'est-à-dire en forme de calotte sphérique concave centrée sur l'axe 202 ; par cette face 254, la paroi 253 est appelée à venir en contact avec la surface 18 de la cornée 2 sans appliquer toutefois à celle-ci de pression. A titre d'exemple non limitatif, la paroi 253 est retenue de façon solidaire mais amovible par engagement périphérique dans une gorge 255 annulaire de révolution autour de l'axe 202, aménagée dans la face 249 à proximité de la face 248 et tournée vers l'axe 202, cette gorge 255 étant délimitée par deux pièces (non référencées) mutuellement vissées du corps 203 afin de permettre de changer la paroi 253.

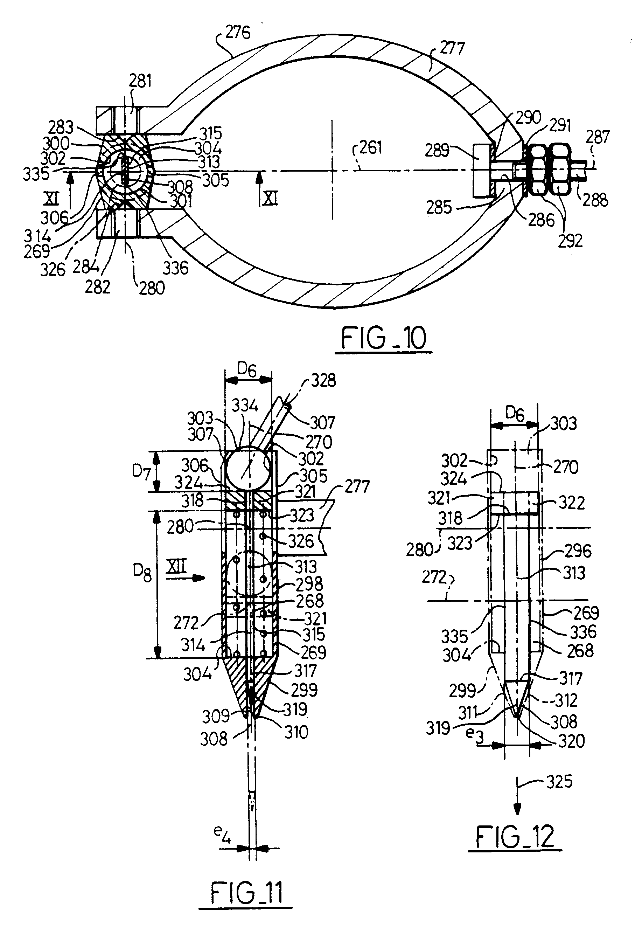

A l'opposé de sa face 254, la paroi 253 présente une face 256 convexe, parallèle à la face 254, et qui délimite ainsi vers la partie de pied 204 la cavité intérieure 257 du corps 203 ; cette cavité 257 est ouverte à l'opposé de la partie de pied 204 du support 201 par un orifice 258 délimité par la face 245 dans le sens d'un éloignement par rapport à l'axe 202 et permettant de voir la paroi 253 à travers la cavité 257 ; pour faciliter un positionnement du kératotome 200 en parfaite coïncidence de son axe 202 avec l'axé visuel, sur la cornée 2 à inciser, cette paroi 253 est avantageusement percée de part en part suivant l'axe 202 d'un trou 259 cylindrique de révolution autour de l'axe 202 et présentant un diamètre (non référencé) aussi faible que possible, par exemple de l'ordre de 0,05 mm ; elle est en outre de préférence largement ajourée afin de permettre au chirurgien de voir la surface 18 de la cornée 2 en cours d'incision à travers l'orifice 258 et la cavité 257 et on a par exemple illustré à cet effet, à la figure 9, sept orifices 260 de grandes dimensions, étant entendu que cet exemple n'est nullement limitatif et que, en particulier, ces orifices 260 pourraient être remplacés par un plus grand nombre d'orifices de plus petites dimensions, donnant à la paroi 253 l'aspect d'une grille ; compte tenu du fait que, lors de l'utilisation du kératotome 200, c'est-à-dire de la rotation du corps 203 par rapport au support 201 fixe par rapport à la cornée 2, la face 254 de la paroi 253 se déplace au contact de la surface 18 de cette cornée, les orifices 260, ou les orifices les remplaçant, et le trou 259 sont délimités par des arêtes adoucies au moins à leur jonction avec la face concave 254 de la paroi 253.

Il en est de même de deux autres trous traversant 262 et 263 aménagés respectivement de part et d'autre de l'axe 202 dans la paroi 253, suivant un même plan moyen 261 fixe par rapport au corps 203 et incluant l'axe 202 et suivant des axes respectifs 264, 265 situés dans ce plan 261 et coupant la face 254, ou encore la surface de référence 210, en des points 266, 267 rigoureusement symétriques l'un de l'autre par rapport à l'axe 202 et mutuellement distants, perpendiculairement à cet axe, d'une valeur égale au diamètre D₂ du cercle 16 à réaliser au départ de l'incision 14 ou 114.

Ces trous 262 et 263, qui présentent perpendiculairement à leur axe respectif une même section rectangulaire, symétrique par rapport au plan 261 et définie par deux faces parallèles à ce plan et deux faces perpendiculaires à ce plan, et sont bordés sur la face 256 de la paroi 253 par des protubérances périphériques respectives 366, 367 dont chacune converge vers celui de ces deux trous 262, 263 qui lui correspond, permettent la traversée de la paroi 253 par un couteau 268 porté par un porte-couteau 269 monté oscillant à l'intérieur de la cavité 257, par rapport au corps 203, pour permettre l'orientation d'un axe 270 commun au couteau 268 et au porte-couteau 269 et déterminant une direction propre de ces derniers, dans le plan moyen 261 fixe par rapport au corps 203, soit suivant l'axe 264, soit suivant l'axe 265, en passant par toutes les orientations intermédiaires ; toutefois, dans l'exemple non limitatif de réalisation d'un kératotome selon l'invention qui a été illustré, ces orientations limites sont seules utilisées pour réaliser, au moyen du couteau 268, une incision 14 ou 114 et, à cet effet, l'axe 264 est parallèle à l'axe 202 et décalé par rapport à celui-ci d'une distance correspondant à la moitié de D₂ alors que l'axe 265 est sécant de l'axe 202 en regard de la face 256 de la paroi 253 et oblique par rapport à l'axe 202, par rapport auquel il diverge en direction de la paroi 253 ; plus précisément. l'axe 265 forme l'angle A, défini en référence à la figure 2, par rapport à un plan 271 perpendiculaire à l'axe 202.

En vue de permettre un tel réglage d'orientation, le porte-couteau 269 est monté tourillonnant sur une chape 273 solidaire du corps 203, à l'intérieur de celui-ci, autour d'un axe 272 perpendiculaire à la fois au plan moyen 261 et à l'axe commun 270 du couteau 268 et du porte-couteau 269, et passant par l'intersection des axes 264 et 265, cet axe 272 étant ainsi décalé à la fois par rapport à l'axe 202 et par rapport à la paroi 253, et ce vers l'intérieur de la cavité 257 au niveau de la face 250 du corps 203 ; par exemple, l'axe 272 est matérialisé par des vis pointeaux qui s'engagent coaxialement dans des paliers coniques du couteau 269, de façon non représentée mais aisément concevable par un Homme du métier, et sont portées par une chape 273 d'une bague annulaire 274, de révolution autour de l'axe 202, insérée dans la cavité 257 du corps 203 dans une position telle qu'elle double la face 249 de celui-ci, avec solidarisation mutuelle par exemple par collage ou goupillage.

En outre sont prévus des moyens 275 de tout type approprié pour faire pivoter de façon réglée le porte-couteau 269, et avec lui le couteau 268, autour de l'axe 272 par rapport au corps 203 de l'une à l'autre de ses orientations correspondant à une coïncidence de leur axe commun 270 respectivement avec l'axe 264 et avec l'axe 265.

Dans l'exemple non limitatif illustré, ces moyens 275 coopèrent, à cet effet, avec le porte-couteau 268 par une pièce en forme de fourche 276 logée dans la cavité 257 du corps 203 et comportant deux bras 277, 278 symétriques l'un de l'autre par rapport au plan moyen 261 et situés suivant un même plan moyen propre 279, perpendiculaire au plan 261, oblique par rapport à l'axe 202 avec variation de son inclinaison par rapport à celui en fonction de l'orientation du porte-couteau 269 autour de l'axe 272 par rapport au corps 203, et se confondant avec le plan de la figure 10. Les deux bras 277 et 278 de la pièce 276 s'articulent sur le porte-couteau 269, en des positions mutuellement symétriques par rapport à la direction moyenne 270 de celui-ci, autour d'un axe 280 situé perpendiculairement au plan 261 et à l'axe 270, dans le plan 279, c'est-à-dire parallèlement à l'axe 272 d'articulation du porte-couteaux 269 par rapport au corps 203, à l'opposé de la paroi 253 par rapport a cet axe 272 quelle que soit l'orientation de l'axe 270 du porte-couteau 269 dans des conditions normales d'utilisation du kératotome 200, c'est-à-dire que cette orientation coïncide avec l'axe 264 ou avec l'axe 265 ou qu'elle soit intermédiaire entre ces deux orientations limites ; à cet effet, par exemple, chacun des bras 277, 278 porte de façon solidaire suivant l'axe 280 une vis pointeau respective 281, 282 qui s'engage coaxialement dans une portée conique respective 283, 284 du porte-couteaux 269, montage auquel est analogue le montage d'articulation du porte-couteau autour de l'axe 272 par rapport à la chape 273 ; naturellement, d'autres montages articulés pourraient être adoptés sans que l'on sorte pour autant du cadre de la présente invention.

A l'opposé de leur articulation autour de l'axe 280 sur le porte-couteau 269, les deux bras 277 et 278 se raccordent mutuellement de façon solidaire, par l'intermédiaire d'une âme 285 percée de part en part d'un alésage 286 cylindrique de révolution autour d'un axe 287 situé à l'intersection des plans 261 et 279 et coupant perpendiculairement l'axe 280. Par cet alésage 286, situé approximativement au niveau de l'orifice 258 alors que l'axe 280 est placé plus près de l'axe 272 que de cet orifice 258, la pièce en forme de fourche 276 reçoit et guide à la rotation relative autour de l'axe 287 une tige filetée 288 présentant entre les deux bras 277 et 278 de la pièce 276, c'est-à-dire du même côté de l'âme 285 que l'axe 280, une tête 289 par laquelle la tige filetée 288 s'appuie sur l'âme 285, suivant l'axe 287, par l'intermédiaire d'une rondelle 290 ; à l'opposé de l'âme 285, la tige filetée 288 s'appuie sur cette dernière, par l'intermédiaire d'une rondelle 291, par un ensemble écrou - contre-écrou 292 qui, sans nuire à la possibilité de rotation de la tige filetée 288 autour de l'axe 287 à l'intérieur de l'alésage 286, s'oppose à tout autre déplacement de la tige filetée 288 par rapport à la pièce 276.

Compte tenu de l'orientation, indiquée plus haut, de la pièce 276, la tige filetée 288 sort de la cavité 257 du corps 203 par l'orifice 258 de celui-ci, à l'opposé de la tête 289 par rapport à l'âme 285 de la pièce 276, du côté de l'axe 202 opposé au côté de celui-ci où se trouve l'axe 272 d'articulation du porte-couteau 269 sur le corps 203, et s'engage par son filetage, à l'extérieur du corps 203, dans un manchon taraudé complémentairement 293 articulé, autour d'un axe 294, sur une chape 295 sortant de la cavité 257 par l'orifice 258 et portée de façon solidaire par une bague 296, annulaire de révolution autour de l'axe 202 et accolée à la face 252 du corps 203à proximité immédiate de l'orifice 258 ; cette bague 296 est solidarisée avec le corps 203 par tout moyen approprié, tel qu'un vissage au moyen de vis radiales par rapport à l'axe 202, et l'axe 294 est orienté perpendiculairement à l'axe 287 et au plan 261, c'est-à-dire parallèlement aux axes 280 et 272, dans le plan 279, en étant situé du côté de l'axe 202 opposé au côté de cet axe où se situe l'axe 272.

On comprend aisément qu'en faisant tourner la tige filetée 288 sur elle-même autour de l'axe 287, dans un sens ou dans l'autre, à l'intérieur du manchon taraudé 293, on puisse à volonté soit rapprocher, soit éloigner l'axe 280 de l'axe 294 et ainsi orienter l'axe 270 du porte-couteau 269 et du couteau 268 par rapport au corps 203 notamment suivant l'axe 264 ou suivant l'axe 265. A cet effet, la tige filetée 287 porte de façon solidaire, mais de préférence réglable, à l'opposé du manchon 293 par rapport à sa tête 289, une mollette 297 avantageusement munie, conjointement avec le manchon 293, de graduations micrométriques en orientation de l'axe 270 du couteau 268 et du porte-couteau 269 par rapport à l'axe 202 ou par rapport au plan 271 perpendiculaire à cet axe 202.

De préférence, les orientations limites dans lesquelles l'axe 270 coïncide respectivement avec l'axe 264 et avec l'axe 265 sont définies par des butées avantageusement réglables ; ainsi, dans l'exemple illustré, dans l'orientation dans laquelle le porte-couteau 269 est illustré en trait plein à la figure 8, à savoir une orientation dans laquelle son axe 270 coïncide avec l'axe 265, le porte-couteau 269 s'appuie sur le corps 203, dans une zone de celui-ci correspondant à sa face 250 et approximativement suivant l'axe 287, contre un ensemble de vis - contre-vis 388 visé dans un alésage taraudé 389 du corps 203 alors que dans l'orientation, illustrée en trait mixte, dans laquelle l'axe 270 coïncide avec l'axe 264, parallèlement à l'axe 202, l'ensemble écrou - contre-écrou 292 s'appuie (de façon non représentée) contre le manchon 293, éventuellement par l'intermédiaire d'une rondelle interchangeable, non représentée, permettant un réglage fin de la butée.

Naturellement, le passage du couteau 268 et du porte-couteau 269 de l'une à l'autre des orientations 264 et 265, si l'on se réfère à leur axe commune 270, nécessite que le porte-couteau 269 soit situé intégralement en retrait vers l'intérieur de la cavité 257, par rapport à la paroi 253, dans ces deux orientations limites ainsi que dans toute orientation intermédiaire, et que le couteau 268 soit également ainsi placé, alors que ce couteau 268 doit également pouvoir être place en saillie par rapport à la face 254 de la paroi 253 une fois que l'une des deux orientations limites est atteinte, afin de permettre l'incision de la cornée.

A cet effet, le couteau 268 et le porte-couteau 269 présentent des formes qui vont être décrites à présent, en référence aux figures 10 à 12, à titre d'exemple non limitatif.

Comme le montre la figure 11, le porte-couteau 269 présente une forme générale oblongue suivant l'axe 270 et comporte deux zones 298, 299 mutuellement adjacentes suivant cet axe. Dans sa zone 298, dans laquelle se situent les deux axes d'articulation 272 et 280, le porte-couteau 269 présente la forme générale d'une paroi tubulaire de révolution autour de l'axe 270, à l'exception de deux zones étroitement localisées autour des axes 272 et 280, dans lesquelles il présente extérieurement des bossages de réception des vis pointeaux matérialisant ces axes, comme on l'a illustré en 300 et 301 à la figure 10 à propos de l'articulation autour de l'axe 280 au moyen des vis pointeaux 280 et 282, un montage analogue étant utilisé pour le montage articulé autour de l'axe 272 ; par contre, intérieurement, le porte-couteau 269 présente dans sa zone 298 une face périphérique intérieure 302 intégralement cylindrique de révolution autour de l'axe 270, entre une extrémité ouverte 303 située entre l'axe 280 et l'orifice 258 du corps 203 dans l'une quelconque des orientations du porte-couteau 269 définies plus haut et une extrémité 304 matérialisée par une face plane perpendiculaire à l'axe 270, à la jonction entre les zones 298 et 299, entre l'axe 272 et la paroi 253 dans lesdites orientations ; de l'extrémité ouverte 303 jusqu'à mi-distance, approximativement, entre les axes 272 et 280, la paroi tubulaire constituant la zone 298 du porte-couteau 269 présente suivant le plan 261, de part et d'autre de l'axe 270, deux fentes 305, 306 permettant le passage d'une tige 307 de moyens micrométriques 308 de réglage de profondeur d'incision, lesquels seront décrits plus loin alors que, pour permettre de réaliser le kératotome 200 sous un encombrement aussi réduit que possible, cette paroi tubulaire est munie à proximité de l'extrémité 303, également suivant le plan 261, d'un biseau 307 permettant d'adopter l'orientation correspondant à une coïncidence de l'axe 270 avec l'axe 265 sans contact direct entre le porte-couteau 269 et le corps 203 comme le montre la figure 8. Dans sa zone 299, le porte-couteau 269 présente extérieurement une forme tronconique de révolution autour de l'axe 270 et convergeant vers la paroi 253 dans les orientations précitées du porte-couteau 269, avec des dimensions telles que quelle que soit l'orientation ainsi présentée par celui-ci, il ne vienne jamais au contact d'une zone quelconque de la paroi 253 ; dans cette zone 299, le porte-couteau 269 est percé d'une fente plate 308 présentant perpendiculairement à l'axe 270 une section rectangulaire, comme le montre la figure 10 ; cette section est définie notamment par deux faces 309 et 310 mutuellement parallèles, symétriques l'une de l'autre par rapport à un plan qui est perpendiculaire au plan 261, inclut l'axe 270 aussi bien que les axes 272 et 280, et dont la trace se confond avec l'axe 270 à la figure 11 et avec l'axe 280 à la figure 10 ; la fente 308 est en outre délimitée par deux faces planes 311, 312 mutuellement parallèles et symétriques l'une de l'autre par rapport au plan 261 comme le montre la figure 12 où l'on a schématisé le porte-couteau 269 en trait mixte ; si D₆ désigne le diamètre de la face 302, les faces 311 et 312 sont ainsi écartées mutuellement, perpendiculairement à l'axe 270, d'une distance e₃ inférieure à D₆ et par exemple de l'ordre de la moitié de D₆ alors que les faces 309 et 310 sont écartées mutuellement, perpendiculairement au plan commun des axes 270, 272, 280 d'une distance e₄ inférieure à e₃, et par exemple de l'ordre de la moitié de e₃ de telle sorte que, notamment, la face d'extrémité 304 borde de toutes parts la fente 308 comme le montre la figure 10.

En correspondance avec cette conformation du porte-couteau 269, le couteau 268 présente la forme générale d'une lame plate 313 présentant perpendiculairement à l'axe 270 une section courante sensiblement identique à celle de la fente 308 de telle sorte que cette dernière puisse guider la lame 313 au coulissement relatif suivant l'axe 270, à l'exclusion de toute autre possibilité de déplacement relatif, par contact glissant des faces 309 à 312 avec des faces planes respectives de la lame 313, à savoir, respectivement, des faces 314 et 315 mutuellement parallèles et symétriques l'une de l'autre par rapport au plan commun des axes 270, 272 et 280, et des faces 335 et 336 mutuellement parallèles et symétriques l'une de l'autre par rapport au plan 261. La lame 313 présente une telle section, de façon constante, entre deux extrémités 317 et 318 tournées respectivement vers la paroi 253 et vers l'orifice 258 dans l'une quelconque des orientations précitées, communes, du porte-couteau 269 et du couteau 268. Vers son extrémité 317, la lame 313 porte de façon solidaire une pointe coupante, par exemple diamantée, 319 qui, dans l'exemple illustré, présente la forme d'une lancette symétrique par rapport au plan commun des axes 270, 272 et 280 et par rapport au plan 261 et se termine en pointe acérée 320 vers la paroi 253, étant entendu que toute autre forme de pointe coupante pourrait être choisie sans que l'on sorte pour autant du cadre de la présente invention. Par son extrémité 318, la lame 313 est solidaire d'un piston 321 présentant une forme générale de révolution autour de l'axe 270, définie par une face périphérique 322 cylindrique de révolution autour de cet axe avec un diamètre sensiblement égal à D₆ de telle sorte que s'établisse un contact glissant, de guidage mutuel au coulissement suivant l'axe 270, entre cette face 322 du piston 321 et la face 302 du porte-couteaux 269 ; le piston 321 est par ailleurs délimité par deux faces planes, perpendiculaires à l'axe 270, à raison d'une face 323 placée en regard de la face d'extrémité 304 et d'une face 324 tournée à l'oppose, c'est-à-dire vers l'orifice 258 du corps 203 dans les orientations de l'axe 270 définies plus haut.

Le couteau 268 et le porte-couteau 269 sont dimensionnés, d'une façon aisément concevable par un Homme du métier, de telle sorte que le couteau 268 puisse occuper notamment une position de repos illustrée en trait plein à la figure 11, à savoir une position dans laquelle la lame 313 est engagée, par une zone adjacente à son extrémité 317, dans la fente 308 à l'intérieur de laquelle la pointe coupante 319 reste cependant totalement escamotée, alors que le piston 321, placé à l'intérieur de la zone 298 du porte-couteau 269 avec contact mutuel des faces 322 et 302, est distant de l'extrémité 303, par sa face 324, d'une distance D₇ voisine de D₆ alors qu'il est distant de la face d'extrémité 304, par sa face 323, d'une distance D₈ largement supérieure à D₇ ; cette distance D₈ est telle que le couteau 268 puisse être déplacé, par coulissement suivant l'axe 270 par rapport au porte-couteau 269, jusqu'à une position de coupe illustrée en trait mixte à la figure 11 et dans laquelle, après rapprochement du piston 321 par rapport à la face d'extrémité 304, la pointe coupante 319 forme une saillie non seulement hors du porte-couteau 269, mais également hors du corps 203, à travers la paroi 253 via l'un des trous 262 et 263 selon que l'axe 270 coïncide avec l'axe 264 ou avec l'axe 265, pour être ainsi placée en saillie par rapport à la face 254 de la paroi 253, c'est-à-dire par rapport a la surface de référence 210, pour pénétrer dans une cornée 2 dont la surface 18 épouse la face 254 et inciser ainsi cette cornée ; on remarque que si l'axe 270 coïncide avec l'axe 264, l'incision est pratiquée parallèlement à l'axe 202, c'est-à-dire sous forme d'un cylindre de révolution autour de cet axe 202 lorsque l'on fait tourner le corps 203 à l'intérieur du support 201 en agissant sur les moyens d'entraînement 232 alors que si l'axe 270 coïncide avec l'axe 265, la pointe coupante 319 diverge par rapport à l'axe 202 dans un sens 325 de l'axe 270 allant de l'extrémité 318 de la lame 313 vers son extrémité 317 si bien que, lors de la rotation du corps 203 autour de l'axe 202 par rapport au support 201, la pointe coupante 319 pratique une incision tronconique, divergeant à partir de la surface 18 de la cornée confondue avec la surface de référence 210 ; toutefois, en raison du positionnement des axes 264 et 265 défini plus haut, l'incision cylindrique et l'incision tronconique ainsi réalisées conservent au niveau de la surface de référence 210, c'est-à-dire au niveau de la surface 18 de la cornée e, un même diamètre égal à D₂.

Les moyens 308 de réglage de profondeur d'incision, évoqués plus haut, sont utilisés pour amener à volonté le couteau 268 dans sa position de coupe, en saillie réglée par rapport a la face 254 de la paroi 253 ou par rapport à la surface de référence 210, ou au contraire ramener le couteau 268 dans sa position de repos dans laquelle sa pointe 308 est escamotée à l'intérieur du porte-couteau 269 et par rapport à la paroi 253 et peuvent présenter toute forme appropriée à cet effet.

Dans l'exemple non limitatif illustré, le mouvement d'escamotage est provoqué automatiquement par un ressort hélicoïdal 326 travaillant à la compression entre la face 323 du piston 321 et la face d'extrémité 304 du porte-couteau 269, autour de la lame 313 mais en retrait vers l'axe 270 par rapport à la face 302 du porte-couteau 269 comme le montrent les figures 10 et 11.

Les moyens 308 de réglage de profondeur d'incision sont alors constitués par des moyens, à simple effet, appliquant au piston 321, par sa face 324, une poussée antagoniste de celle du ressort 326 mais il est bien entendu que l'on ne sortirait pas du cadre de la présente invention en utilisant des moyens de réglage de profondeur d'incision à double effet, notamment en l'absence de ressort 326, pour commander de façon positive non seulement le mouvement du couteau 268 vers sa position de coupe mais également son mouvement vers la position de repos, ou encore tout autre moyen permettant, par translation du couteau 268 suivant l'axe 270 par rapport au porte-couteau 260, de former une saillie réglée de la pointe coupante 308 par rapport à la face 254 de la paroi 253 et par rapport à la surface de référence 210.

Dans l'exemple illustré, les moyens de réglage de profondeur d'incision 308 sont de nature micrométrique et comportent un manchon 327 percé, suivant un axe propre 328, d'un alésage taraudé 331 et monté à pivotement, par rapport au corps 303, autour d'un axe 329 sur une chape 330 solidaire du corps 203 dans une position opposée à celle de la chape 295 par rapport à l'axe 202 ; la chape 330 forme une saillie hors du corps 203, par l'orifice 258 de celui-ci, de telle sorte que l'axe 329 se situe approximativement dans un même plan (non illustré) perpendiculaire à l'axe 202 que l'axe 294, auquel l'axe 329 est parallèle de telle sorte que, lors du pivotement du manchon 327 autour de l'axe 329 par rapport à la chape 330, son axe 328 reste en permanence dans le plan 261 ; en outre, l'axe 329 est disposé de telle sorte que l'axe 280 se situe, par rapport à lui, sensiblement à la même distance lorsque l'axe 270 du couteau 268 et du porte-couteau 269 coïncide avec l'axe 264 ou coïncide avec l'axe 265.

La tige 307 précitée, rectiligne, traverse de part en part, coaxialement, le manchon 327 et coopère avec le taraudage de son alésage 331 par un filetage qui en est complémentaire de telle sorte qu'une rotation de la tige 307 autour de l'axe 328 par rapport au manchon 327 provoque une translation relative parallèlement à l'axe 328.

Une telle rotation relative peut être provoquée à volonté au moyen d'une mollette 332 que la tige 307 porte d'un côté du manchon 327, de façon solidaire mais avantageusement réglable suivant l'axe 328 comme on l'a dit plus haut à propos de la mollette 297 et de la tige filetée 288 des moyens 275 de réglage de l'orientation du porte-couteau 269 et du couteau 268 ; comme la mollette 297 et le manchon 293 avec lequel coopère la tige 288, la mollette 332 et le manchon 327 portent conjointement des graduations micrométriques 333 ; ces graduations 333, avantageusement exprimées en profondeur d'incision, c'est-à-dire en valeur de la saillie formée par la pointe coupante 308 du couteau 268, suivant sa direction propre 270, par rapport à la face 254 de la paroi 253 et par rapport à la surface de référence 210, sont de préférence subdivisées, sur le manchon 327, en deux zones faciles à distinguer mutuellement, par exemple par un choix de couleurs différentes, et correspondant aux graduations utilisables lorsque l'axe 270 du couteau 268 et du porte-couteau 269 coïncide respectivement avec l'axe 264 et avec l'axe 265.

Naturellement, la mollette 332 des moyens de réglage de profondeur d'incision 308 est disposée à l'extérieur du corps 203 dans lequel la tige 307 pénètre, à l'opposé du manchon 327 par rapport à la mollette 332, via l'orifice 258 pour coopérer avec le piston 321 du couteau 268 à l'intérieur du porte-couteau 269.

A cet effet, la tige 307 porte de façon solidaire, à son extrémité opposée à son extrémité portant la mollette 332 par rapport au manchon 327, une rotule sphérique 334 d'un diamètre sensiblement égal à D₆, laquelle rotule 334 est engagée à l'intérieur de la zone 298 du porte-couteau 269, en appui contre la face 324 du piston 321 et en contact avec la face 302 de façon à être guidée par cette dernière à la translation relative suivant l'axe 270 à l'exclusion de toute autre possibilité de déplacement relatif ; des moyens de butée, non représentés mais aisément concevables par un Homme du métier, peuvent être prévus pour éviter tout échappement de la rotule 334 hors de la zone 298 du porte-couteau 269, par l'extrémité 303, bien que de tels moyens ne soient pas indispensables.

Naturellement, pour que la tige 307 applique au piston 321, par sa rotule 334, une poussée antagoniste à celle du ressort 326 quelle que soit l'orientation de l'axe 270, c'est-à-dire que celui-ci soit orienté suivant l'axe 264 ou suivant l'axe 265 ou dans une orientation intermédiaire, l'axe 329 est placé suffisamment loin de l'axe 272 pour que la tige 307, par son axe 328, et l'ensemble couteau 268 - porte-couteau 269, par son axe 270, forment en permanence, entre eux, un angle C largement supérieur à 90° aussi bien dans les deux orientations limites de coïncidence avec les axes 264 et 265 que dans les orientations intermédiaires entre ces deux orientations limites, lors du passage de l'une à l'autre ; dans l'exemple non limitatif illustré, la valeur de l'angle C est de l'ordre de 140° dans les deux orientations limites et varie entre cette valeur et 180° lors du passage de l'une à l'autre de ces orientations limites ainsi, la tige 307 applique en permanence au couteau 268, par la rotule 334, une poussée dans le sens 325.

On va à présent décrire le fonctionnement du kératotome 200 qui vient d'être décrit, en référence à la réalisation d'une incision du type illustré en 114 à la figure 5.

Dans un état initial du kératotome 200, le couteau 268 est placé en position de repos et l'ensemble couteau 268 - porte-couteau 269 est placé dans une orientation telle que son axe 270 coïncide avec l'axe 265, comme on l'a illustré en trait mixte à la figure 8, par un positionnement approprié des mollettes 332 et 297.

En se guidant au moyen du trou 259, le chirurgien place alors le kératotome 200, par sa partie de pied 204, sur la surface 18 de la cornée 2 à inciser, en plaçant l'axe 202 en coïncidence avec l'axe visuel ; les moyens d'aspiration 211 sont alors mis en route, pour mettre la cavité 206 en dépression et ainsi, d'une part, immobiliser le support 201 sur la cornée par appui sur les portées 208 et 209 et, d'autre part et du fait de cet appui, donner à la surface 18 de la cornée 2 une forme coïncidant avec la surface de référence 210, c'est-à-dire une forme telle qu'elle épouse, toutefois sans pression de contact mutuel, la face 254 de la paroi 253.

Alors, en agissant sur la mollette 332 mais en conservant la coïncidence entre l'axe 270 et l'axe 264, on amène le couteau 268 dans une position de coupe telle qu'il forme par sa pointe coupante 319 une saillie par rapport à la face 254 de la paroi 253 et par rapport à la surface de référence 210 ; au cours du passage à cette position, la pointe coupante 319 pénètre dans la cornée 2, d'une profondeur choisie, réglée par lecture de la graduation 333 et par exemple directement égale à e₂, soit de l'ordre de 50 µm.

Alors, en agissant sur le bouton 244 ou au moyen du moteur le remplaçant, le chirurgien provoque une rotation du corps 203 autour de l'axe 202 par rapport au support 201, sur au moins 360° pour former la zone cylindrique 115 de l'incision 114 à réaliser.

Ensuite, après avoir arrêté la rotation du corps 203 par rapport au support 201, et en agissant sur la mollette 332, le chirurgien ramène le couteau 268 à sa position de repos, c'est-à-dire l'escamote à l'intérieur du porte-couteau 269 puis, en agissant sur la mollette 297, amène l'axe 270 de ce dernier et du couteau 268 en coïncidence avec l'axe 265.

En agissant à nouveau sur la mollette 332, le chirurgien ressort alors le couteau 268 du porte-couteau 269 et, en lui faisant traverser la paroi 253 par le trou 263, l'amène en saillie suivant l'axe 265 par rapport à la face 254 et à la surface de référence 210, et ceci d'une valeur qu'il règle en lisant la graduation 333 ; on remarquera que lors de ce mouvement, la pointe coupante 319 revient à l'intérieur de la zone 315, déjà réalisée, de l'incision 114 à réaliser.

Ensuite, en agissant manuellement sur le bouton 244 ou en mettant en service le moteur le remplaçant, le chirurgien provoque une nouvelle rotation du corps 203 autour de l'axe 202 par rapport au support 201, sur au moins 360°, ce qui forme une partie de la zone 117, directement adjacente à la zone 115, de l'incision 114 à réaliser.

Ensuite, en agissant à nouveau sur la mollette 332 sans agir sur la mollette 297, après arrêt de la rotation, le chirurgien augmente la valeur de la saillie de la pointe coupante 319 par rapport à la face 254 de la paroi 253 et par rapport à la surface de référence 210, ce qui fait pénétrer plus profondément la pointe coupante 319 dans la cornée 2, puis il provoque une nouvelle rotation du corps 203 autour de l'axe 202 par rapport au support 201, sur au moins 360°, pour former une nouvelle partie de la zone 117 de l'incision 114, et ces opérations sont répétées jusqu'à ce que l'intégralité de la zone 117, jusqu'au cercle 17, soit ainsi réalisée.

Alors, en agissant sur la mollette 332, après arrêt de la rotation, le chirurgien ramène le couteau 268 en position de repos, arrête les moyens d'aspiration 211, casse le vide dans la cavité 206 pour faire cesser la solidarisation entre le kératotome 200 et la cornée 2, et enfin éloigne le kératotome 200 ; la mise en place de la lentille 101 peut ensuite être effectuée comme on l'a indiqué plus haut.

Lorsqu'il s'agit d'effectuer une incision 14 du type illustré à la figure 2, il est possible de n'utiliser que l'orientation du porte-couteau 269 et du couteau 268 correspondant à une coïncidence des axes 270 et 265, d'une façon aisément déductible de ce qui vient d'être décrit. Toutefois, on obtient une plus grande précision dans la conformation de l'incision en commençant néanmoins cette dernière en orientant l'axe 270 suivant l'axe 264 comme on l'a décrit plus haut, pour inciser d'abord la cornée en cylindre, sur 360°, sur une profondeur qui peut être bien inférieure à la valeur de 50 µm indiquée ci-dessus, c'est-à-dire peut rester négligeable par rapport aux autres dimensions de l'incision tout en facilitant la pénétration initiale de la pointe coupante 319 dans la cornée 2 et en assurant ainsi une parfaite circularité au cercle 16 par lequel l'incision 14 débouche à la surface 18 de la cornée 2 ; la valeur de la dimension e₂ en ce qui concerne notamment la face 103 de la lentille 101 (figures 5, 6, 7) et la zone 115 de l'incision 114 (figure 5) est essentiellement dictée par un tel soucis de précision de l'incision et peut de ce fait rester bien inférieure à la dimension que présentent, parallèlement à l'axe visuel, les incisions réalisées dans la cornée pour recevoir les lentilles jusqu'à présent utilisées en épikératophakie.

On comprendra aisément que le mode de réalisation du kératotome 200 selon l'invention qui vient d'être décrit ne constitue qu'un exemple non limitatif par rapport auquel un Homme du métier pourra prévoir de nombreuses variantes sans sortir pour autant du cadre de la présente invention selon les revendications, notamment dans le but d'autoriser un positionnement du couteau et du porte-couteau non seulement dans deux orientations limites prédéterminées, mais également dans toute autre orientation intermédiaire choisie à volonté, tout en conservant une identité de positionnement, par rapport à l'axe 202 de rotation du corps 203 par rapport au support 201, de l'intersection de la pointe coupante 319 avec la surface de référence 210, c'est-à-dire avec la face 254 de la paroi 253 si l'on se réfère à l'exemple illustré.

## Revendications

1. Lentille pour épikératophakie, comprenant :
- une zone optique (3) présentant une forme lenticulaire d'axe optique (5) déterminé et délimitée par des faces respectivement antérieure (6), convexe, et postérieure (7), concave, de géométrie respective déterminée d'une part en fonction de la possibilité d'appliquer intimement la face postérieure de la zone optique (3) contre une cornée (2) et d'autre part en fonction de caractéristiques optiques prédéterminées, et
- une zone d'ancrage (4) présentant une forme annulaire de révolution autour de l'axe optique (5) et bordant la zone optique (3) dans le sens d'un éloignement par rapport à l'axe optique (5), cette zone d'ancrage (4) étant notamment délimitée par une face postérieure (11) qui se raccorde à la face postérieure (7) de la zone optique (3) et forme une saillie par rapport à la face postérieure (7) de la zone optique (3) et par une face antérieure (10) qui se raccorde à la face antérieure (6) de la zone optique (3) et est disposée en retrait par rapport à la face antérieure (6) de la zone optique (3),
caractérisée en ce que les faces respectivement antérieure (10) et postérieure (11) de la zone d'ancrage convergent mutuellement dans le sens d'un éloignement par rapport à l'axe optique (5), de façon à déterminer un amincissement progressif de la zone d'ancrage (6) à partir de la zone optique (3) et jusqu'à un bord périphérique (12, 13) de la lentille (1, 101), pour permettre d'insérer la zone d'ancrage (4) dans une incision annulaire (14, 114) de la cornée (2).

2. Lentille selon la revendication 1, caractérisée en ce que, en référence à un plan perpendiculaire à l'axe optique (5), la face antérieure (10) de la zone d'ancrage (4) présente une inclinaison (B) sensiblement inférieure à 90°.

3. Lentille selon la revendication 2, caractérisée en ce que, en référence audit plan perpendiculaire à l'axe optique (5), la face postérieure (11) de la zone d'ancrage (4) présente une inclinaison (A) comprise entre 40° et 45°, et notamment de 42°, et la face antérieure (10) de la zone d'ancrage (4) présente une inclinaison (B) comprise entre 50° et 55°.

4. Lentille selon l'une quelconque des revendications 1 à 3, caractérisée en ce qu'elle est réalisée à partir d'au moins un polymère souple et bio-compatible choisi parmi les polymères naturels et les polymères de synthèse.

5. Lentille selon l'une quelconque des revendications 1 à 4, caractérisée en ce que les faces antérieure (10) et postérieure (11) de la zone d'ancrage (4) présentent approximativement la même forme et les mêmes dimensions (1) lorsqu'elles sont vues en coupe par un quelconque demi-plan limité par l'axe optique (5).

6. Lentille selon l'une quelconque des revendications 1 à 5, caractérisée en ce que les faces (10) antérieure et postérieure (11) de la zone d'ancrage (4) sont approximativement tronconiques de révolution autour de l'axe optique (5).

7. Lentille selon l'une quelconque des revendications 1 à 6, caractérisée en ce que les faces antérieure (10)) et postérieure (11) de la zone d'ancrage (4) se rejoignent mutuellement, sur le bord périphérique (12) de la lentille (1, 101), en une arête circulaire (12).

8. Lentille selon l'une quelconque des revendications 1 à 6, caractérisée en ce que les faces antérieure (10) et postérieure (11) de la zone d'ancrage (4) sont raccordées mutuellement, sur le bord périphérique (112) de la lentille (1, 101), par une face périphérique (13) de révolution autour de l'axe optique (5).

9. Lentille selon la revendication 8, caractérisée en ce que la face périphérique (13) est cylindrique de révolution autour de l'axe optique (5).

10. Lentille selon l'une quelconque des revendications 1 à 9, caractérisée en ce que la face antérieure (6) de la zone optique (3) présente la forme d'une calotte sphérique centrée sur l'axe optique (5).

11. Lentille selon l'une quelconque des revendications 1 à 10, caractérisée en ce que la face antérieure (10) de la zone d'ancrage (4) se raccorde directement à la face antérieure (6) de la zone optique (3).

12. Lentille selon l'une quelconque des revendications 1 à 10, caractérisée en ce que la face antérieure (10) de la zone d'ancrage se raccorde à la face antérieure (6) de la zone optique (3) par l'intermédiaire d'un décrochement périphérique (8) de celle-ci, lequel est de révolution autour de l'axe optique (5).

13. Lentille selon la revendication 12, caractérisée en ce que le décrochement périphérique (8) est cylindrique de révolution autour de l'axe optique (5).

14. Lentille selon l'une quelconque des revendications 1 à 13, caractérisée en ce que la face postérieure (7) de la zone optique (3) présente la forme d'une calotte sphérique centrée sur l'axe optique (5).

15. Lentille selon l'une quelconque des revendications 1 à 13, caractérisée en ce que la face postérieure (7) de la zone optique (3) présente une forme asphérique.

16. Lentille selon la revendication 15, caractérisée en ce qu'elle présente un repère (21) en orientation angulaire autour de l'axe optique (5).

17. Lentille selon l'une quelconque des revendications 1 à 16, caractérisée en ce que la face postérieure (11) de la zone d'ancrage (4) se raccorde directement à la face postérieure (7) de la zone optique (3).

18. Lentille selon l'une quelconque des revendications 1 à 16, caractérisée en ce que la face postérieure (11) de la zone d'ancrage (4) se raccorde à la face postérieure (7) de la zone optique (3) par l'intermédiaire d'un épaulement périphérique (8) de celle-ci, lequel est de révolution autour de l'axe optique (5).

19. Lentille selon la revendication 18, caractérisée en ce que l'épaulement périphérique (8) est cylindrique de révolution autour de l'axe optique (5).

20. Lentille selon l'une quelconque des revendications 1 à 19, caractérisée en ce que la zone d'ancrage (4) présente au moins une entaille (21) débouchant dans le bord périphérique (12, 13) de la lentille (1, 101) et dans des zones des faces antérieure (10) et postérieure (11) de la zone d'ancrage (4) voisines du bord périphérique (12, 13) de la lentille (1, 101).

21. Kératotome destiné à la réalisation d'une incision annulaire dans une cornée et comportant à cet effet :
- un support extérieur tubulaire (201) formant enveloppe, présentant un axe déterminé (202) et comportant, pour son application sur une cornée, une partie de pied (204) annulaire de révolution autour de l'axe (202) du support (201) et délimitant une surface géométrique de référence (210) définie comme la forme que présente la cornée lorsque le support (201) est appliqué sur celle-ci par sa partie de pied (204),
- un corps intérieur tubulaire (203) disposé coaxialement à l'intérieur du support (201), en retrait vers l'intérieur du support (201) par rapport à la surface de référence (210),
- des moyens (214, 218, 222, 224, 225) de guidage du corps à la rotation autour de l'axe (202) du support (201) par rapport au support (201), sur au moins 360°,
- des moyens (232) d'entraînement du corps (203) à la rotation autour de l'axe (202) du support (201) par rapport au support (201), sur au moins 360°,
- un couteau (268) disposé à l'intérieur du corps (203), suivant une direction propre (270), et présentant dans un sens déterminé (325) de sa direction propre (270) une pointe coupante (319),
- des moyens (269, 275, 308) de liaison entre le couteau (268) et le corps (203), comportant eux-mêmes des moyens (308) pour déplacer de façon réglée le couteau (268) en translation suivant sa direction propre (270), par rapport au corps (203), entre une position de repos, dans laquelle il est placé en retrait vers l'intérieur du corps (203) par rapport à la surface de référence (210) et dans laquelle la pointe (319) est tournée vers ladite surface de référence (210), et une position de coupe, dans laquelle la pointe (319) forme une saillie à l'extérieur du corps (203) par rapport à la surface de référence (210), de façon décalée par rapport à l'axe (202) du support (203),
caractérisé en ce que, en vue de la réalisation d'une incision destinée à recevoir la zone d'ancrage d'une lentille selon l'une quelconque des revendications 1 à 20, les moyens (269, 275, 308) de liaison entre le couteau (268) et le corps (203) comportent en outre des moyens (275) pour déplacer de façon réglée le couteau (268) en orientation de sa direction propre (270) par rapport au corps (203), entre plusieurs orientations déterminées dont une première orientation (264) dans laquelle sa direction propre (270) est parallèle à l'axe (202) du support (201) et une deuxième orientation (265) dans laquelle sa direction propre (270) est sécante de l'axe (202) du support (201) et oblique par rapport à cet axe (202) de telle sorte que, au moins en position de coupe, la pointe (319) du couteau (268) diverge dans ledit sens (325) par rapport à l'axe (202), en conservant dans lesdites orientations (264, 265) un écartement sensiblement identique entre l'axe (202) du support (201) et une zone (262, 263) dans laquelle la pointe (319) du couteau (268) traverse la surface de référence (210) en position de coupe.

22. Kératotome selon la revendication 21, caractérisé en ce que lesdites orientations déterminées (264, 265) sont au nombre de deux, a raison desdites première et deuxième orientations (264, 265).

23. Kératotome selon la revendication 22, caractérisé en ce que la pointe (319) du couteau (268) traverse la surface de référence (210), en position de coupe, dans deux zones (262, 263) diamétralement opposées par rapport à l'axe (202) du support (201) respectivement dans l'une et l'autre desdites première et deuxième orientations (264, 265).

24. Kératotome selon l'une quelconque des revendications 21 à 23, caractérisé en ce qu'il comporte à l'intérieur de la partie de pied (204) une paroi (253) solidaire du corps (203) et présentant vers l'extérieur du corps (203) une face coïncidant avec la surface de référence (210), ladite paroi (253) présentant des moyens (262, 263) de passage du couteau (268) en position de coupe.

25. Kératotome selon la revendication 24, caractérisé en ce que ladite paroi (253) comporte des moyens (259, 260) de vision à travers elle et/ou de repérage de positionnement par rapport à la cornée.

26. Kératotome selon la revendication 23 et l'une quelconque des revendications 24 et 25, caractérisé en ce que les moyens de passage (262, 263) comportent deux trous (262, 263).

27. Kératotome selon l'une quelconque des revendications 23 et 26, caractérisé en ce que les moyens (269, 275, 308) de liaison entre le couteau (268) et le corps (203) comportent
- un porte-couteau (269), comportant des moyens (302, 308) de réception du couteau (268) et de guidage de celui à la translation relative suivant sa direction propre (270), et placé en retrait vers l'intérieur du corps (203) par rapport à la surface de référence (210),
- des moyens (273) d'articulation du porte-couteau (269) sur le corps (203), autour d'un axe déterminé (272) d'articulation décalé par rapport à l'axe du support (201), en retrait vers l'intérieur du corps (203) par rapport à la surface de référence (210) et perpendiculaire à la direction propre (270) du couteau (268) et à un plan (261) incluant l'axe (202) du support (201),
- des moyens (275) pour faire pivoter de façon réglée le porte-couteau (269) autour de l'axe d'articulation (272), par rapport au corps (203), de l'une à l'autre de deux orientations (264, 265) correspondant respectivement auxdites première et deuxième orientations (264, 265),
- des moyens (308) pour déplacer de façon réglée le couteau (268) en translation par rapport au porte-couteau (269) entre des positions correspondant, pour l'une et l'autre desdites orientations (264, 265) du porte-couteau (269) par rapport au corps (203), à une position de repos et à une position de coupe.

28. Kératotome selon la revendication 27, caractérisé en ce qu'il comporte des moyens (288, 292, 293) de butée du porte-couteau (269) par rapport au corps (203) respectivement dans l'une et/ou l'autre desdites orientations (264, 265) du porte-couteau (269) par rapport au corps (203).

29. Kératotome selon la revendication 28, caractérisé en ce que les moyens de butée (288, 292, 293) sont réglables.

30. Kératotome selon l'une quelconque des revendications 27 à 29, caractérisé en ce que les moyens (308) pour déplacer de façon réglée le couteau (268) en translation par rapport au porte-couteau (269) sont des moyens de réglage micrométrique (308).

31. Kératotome selon la revendication 30, caractérisé en ce que les moyens de réglage micrométrique (308) sont articulés sur le corps (203) autour d'un axe (329) parallèle à l'axe (272) d'articulation du porte-couteau (269) sur le corps (203), à l'opposé de la surface de référence (210) par rapport à l'axe d'articulation (272) du porte-couteau (269) sur le corps (203), et coopèrent avec le couteau (268) par une poussée appliquée à celui-ci dans ledit sens déterminé (325) de sa direction propre (270), et en ce que le porte-couteau (269) comporte des moyens (326) de sollicitation élastique du couteau (268) en sens opposé audit sens déterminé (325).

32. Kératotome selon l'une quelconque des revendications 21 à 31, caractérisé en ce qu'il comporte des moyens d'indexage de la position angulaire du corps (203) par rapport au support (201) autour de l'axe (202) du support (201).

33. Kératotome selon l'une quelconque des revendications 21 à 32, caractérisé en ce que la partie de pied (204) délimite une cavité annulaire (206) ouverte autour de la surface de référence (210), et en ce que sont prévus des moyens (213) pour mettre la cavité annulaire (206) en dépression.

## Claims

1. A lense for epikeratophakia, comprising :
- an optical zone (3) which has a lenticular shape of predetermined optical axis (5) and is bounded by a convex front face (6) and a concave rear face (7) whose respective geometries are determined on the one hand in relation to the possibility of applying the rear face of the optical zone (3) closely fitting against a cornea (2) and on the other hand in relation to predetermined optical characteristics, and
- an anchoring zone (4) having an annular shape of revolution around the optical axis (5) and edging the optical zone (3) in the direction away from the optical axis (5), wherein the anchoring zone (4) is bounded notably by a rear face (11) which is connected to the rear face (7) of the optical zone (3) and projects from the rear face (7) of the optical zone (3) and by a front face (10) which is connected to the front face (6) of the optical zone (3) and is set back from the front face (6) of the optical zone (3),
characterized in that the front face (10) and the rear face (11) of the anchoring zone converge mutually in the direction away from the optical axis (5) so as to produce a progressive thinning of the anchoring zone (6) from the optical zone (3) as far as a peripheral edge (12, 13) of the lense (1, 101), in order to allow an insertion of the anchoring zone (4) into an annular incision (14, 114) in the cornea (2).

2. A lense according to claim 1, characterized in that the front face (10) of the anchoring zone (4) has an inclinaison (B) substantially less than 90°, with respect to a plane perpendicular to the optical axis (5).

3. A lense according to claim 2, characterized in that the rear face (11) of the anchoring zone (4) has an inclinaison (A) of between about 40° and about 45°, more particularly 42°, and the front face (10) of the anchoring zone (4) has an inclination (B) of between about 50° and about 55°, with respect to said plane perpendicular to the optical axis(5).

4. A lense according to any of Claims 1 to 3, characterized in that it is produced from at least one flexible and biocompatible polymer selected from natural polymers and synthetic polymers.

5. A lense according to any of Claims 1 to 4, characterized in that the front face (10) and the rear face (11) of the anchoring zone (4) have substantially the same shape and the same dimensions when they are viewed in section along any half-plane bounded by the optical axis (5).

6. A lense according to any of Claims 1 to 5, characterized that the front face (10) and rear face (11) of the anchoring zone (4) are substantially frusto-conical faces of revolution around the optical axis (5).

7. A lense according to any of Claims 1 to 6, characterized in that the front face (10) and rear face (11) of the anchoring zone (4) join one another at the peripheral edge (12) of the lense (1, 101) in a circular edge (12).

8. A lense according to any of Claims 1 to 6, characterized in that the front face (10) and rear face (11) of the anchoring zone (4) are connected to one another at the peripheral edge (112) of the lense (1, 101) via a peripheral face (13) of revolution around the optical axis (5).

9. A lense according to Claim 8, characterized in that the peripheral face (13) is a cylindrical face of revolution around the optical axis (5).

10. A lense according to any of Claims 1 to 9, characterized in that the front face (6) of the optical zone (3) takes the form of a spherical cap centred on the optical axis (5).

11. A lense according to any of Claims 1 to 10, characterized in that the front face (10) of the anchoring zone (4) is directly connected to the front face (6) of the optical zone (3).

12. A lense according to any of Claims 1 to 10, characterized in that the front face (10) of the anchoring zone is connected to the front face (6) of the optical zone (3) via a peripheral recess (8) thereof of revolution around the optical axis (5).

13. A lense according to Claim 12, characterized in that the peripheral recess (8) is a cylindrical one of revolution around the optical axis (5).

14. A lense according to any of Claims 1 to 13, characterized in that the rear face (7) of the optical zone (3) takes the form of a spherical cap centred on the optical axis (5).

15. A lense according to any of Claims 1 to 13, characterized in that the rear face (7) of the optical zone (3) has a non-spherical shape.

16. A lense according to Claim 15, characterized in that it has a marking (21) in angular orientation around the optical axis (5).

17. A lense according to any of Claims 1 to 16, characterized in that the rear face (11) of the anchoring zone (4) is directly connected to the rear face (7) of the optical zone (3).

18. A lense according to any of Claims 1 to 16, characterized in that the rear face (11) of the anchoring zone (4) is connected to the rear face (7) of the optical zone (3) via a peripheral shoulder (9) of revolution around the optical axis (5).

19. A lense according to Claim 18, characterized in that the peripheral shoulder (9) is a cylindrical shoulder of revolution around the optical axis (5).

20. A lense according to any of Claims 1 to 19, characterized in that the anchoring zone (4) has at least one notch (21) opening into the peripheral edge (12, 13) of the lense (1, 101) and in zones of the front face (10) and rear face (11) of the anchoring zone (4) close to the peripheral edge (12, 13) of the lense (1, 101).

21. A keratotome for making an annular incision in a cornea and comprising to that end:
- an outer tubular support (201) forming an envelope, having a predetermined axis (202) and comprising for its application to a cornea, an annular base portion (204) of revolution around the axis (202) of the support (201) and bounding a geometrical reference surface (210) defined as the shape which the cornea has when the support (201) is applied thereto via its base portion (204),
- an inner tubular member (203) disposed coaxially with the inside of the support (201) and withdrawn towards the inside of the support (201) in relation to the reference surface (210),
- means (214, 218, 222, 224, 225) for guiding the body in rotation around the axis (202) of the support (201) in relation to the support (201), through at least 360°,
- means (232) for driving the body (203) in rotation around the axis (202) of the support (201) in relation to the support (201) through at least 360°,
- a blade (268) disposed inside the body (203) in a direction of its own (270) and having in a predetermined sense (325) of its own direction (271) a cutting tip (319),
- means (269, 275, 308) for connection between the blade (268) and the body (203) which comprise means (308) for displacing the blade (268) in a controlled manner in translation along its own direction (271) in relation to the body (203), between an inoperative position, in which the blade is withdrawn towards the inside of the body (203) in relation to the reference surface (210) and in which the tip (319) is turned towards said reference surface (210), and a cutting position in which the tip (319) projects outside the body (203) in relation to the reference surface (210) in a manner offset in relation to the axis (202) of the support (203),
characterized in that with a view to making an incision adapted to receive the anchoring zone of a lense according to any of Claims 1 to 20, the means (269, 275, 308) of connection between the blade (268) and the body (203) also comprise means (275) for displacing in a controlled manner the blade (268) in the orientation of its own direction (270) in relation to the body (203), between a number of predetermined orientations, including a first orientation (264) in which its own direction (270) is parallel with the axis (202) of the support (201), and a second orientation (265) in which its own direction (270) intersects the axis (202) of the support (201) and is so inclined in relation to the axis (202) that, at least in the cutting position, the tip (319) of the blade (268) diverges in said direction (325) in relation to the axis (202), while maintaining in said orientations (264, 265) a substantially identical distance between the axis (202) of the support (201) and a zone (262, 263) in which the tip (319) of the blade (268) crosses the reference surface (210) in the cutting position.

22. A keratotome according to Claim 21, characterized in that said predetermined orientations (264, 265) are two in number, namely said first and second orientations (264, 265).

23. A keratotome according to Claim 22, characterized in that the tip (319) of the blade (268) crosses the reference surface (210) in the cutting position in two zones (262, 263) which are diametrically opposed in relation to the axis (202) of the support (201), in one and the other of said first and second orientations (264, 265) respectively.

24. A lense according to any of Claims 21 to 23, characterized in that it comprises inside the base portion (204) a wall (253) rigidly connected to the body (203) and having towards the outside of the body (203) a face coinciding with the reference surface (210), said wall (253) having means (262, 263) for the passage of the blade (268) into the cutting position.

25. A keratotome according to Claim 24, characterized in that said wall (253) comprises means (259, 260) for seeing through the wall and/or for indicating the positioning in relation to the cornea.

26. A keratotome according to Claim 23 and either of Claims 24 and 25, characterized in that the means (262) of passage comprise two holes (262, 263).

27. A keratotome according to any of Claims 23 to 26, characterized in that the means (269, 275, 308) of connection between the blade (268) and the body (203) comprise
- a blade carrier (269) comprising means (302, 308) for receiving the blade (268) and guiding the blade in relative translation along its own direction (270), the blade carrier (269) being disposed withdrawn towards the inside of the body (203) in relation to the reference surface (210),
- means (273) for articulating the blade carrier (269) on the body (203) around a predetermined axis (272) of articulation which is offset in relation to the axis of the support (201), withdrawn towards the inside of the body (203) in relation to the reference surface (210) and perpendicular to the own direction (270) of the blade (268) and to a plane (261) including the axis (202) of the support (201),
- means (275) for pivoting in a controlled manner the blade carrier (269) around the axis (272) of articulation in relation to the body (203), from one to the other of two orientations (264, 265) corresponding to said first and second orientations (264, 265) respectively,
- means (308) for displacing in a controlled manner the blade (268) in relation to the blade carrier (269) between positions corresponding, for one and the other of said orientations (264, 265) of the blade carrier (269) in relation to the body (203), to an inoperative position and a cutting position.

28. A keratotome according to Claim 27, characterized in that it comprises means (288, 292, 293) of abutment of the blade carrier (269) with the body (203) in one and/or the other of said orientations (264, 265) of the blade carrier (269) in relation to the body (203) respectively.

29. A keratotome according to Claim 28, characterized in that the abutment means (288, 292, 293) are adjustable.

30. A keratotome according to any of Claims 27 to 29, characterized in that the means (308) for controllably displacing the blade (268) in translation with respect to the blade carrier (269) are micrometric adjustment means (308).

31. A keratotome according to Claim 30, characterized in that the micrometric control means (308) are articulated to the body (203) around an axis (329) parallel to the axis (272) of articulation of the blade carrier (269) to the body (203), opposite the reference surface (210) in relation to the axis (272) of articulation of the blade carrier (269) to the body (203), and cooperate with the blade (268) by a thrust applied thereto in said predetermined sense (325) of its own direction (270), the blade carrier (269) comprising means (326) for resiliently urging the blade (268) in the sense opposite from said predetermined sense (325).

32. A keratotome according to any of Claims 21 to 31, characterized in that it comprises means for indexing the angular position of the body (203) in relation to the support (201) around the axis (202) of the support (201).

33. A keratotome according to any of Claims 21 to 32, characterized in that the base portion (204) bounds an annular cavity (206) open around the reference surface (210), and means (213) are provided to put the annular cavity (206) under negative pressure.

## Patentansprüche

1. Linse für die Epikeratophakie, mit:
- einem optischen Bereich (3), der eine linsenartige Form mit festgelegter optischer Achse (5) zeigt und durch jeweilige vordere (6) konvexe und hintere (7) konkave Flächen begrenzt ist, mit jeweilig festgelegter Geometrie, einerseits in Abhängigkeit von der Möglichkeit, die hintere Fläche des optischen Bereichs (3) innig an eine Hornhaut (2) zu legen und andererseits in Abhängigkeit von vorbestimmten optischen Eigenschaften, und
- einem Verankerungsbereich (4), der eine ringartige Form im Umlauf um die optische Achse (5) zeigt und an den optischen Bereich (3) angrenzt, in dem Sinne einer Entfernung in bezug auf die optische Achse (5), wobei dieser Verankerungsbereich (4) insbesondere durch eine hintere Fläche (11), die sich an die hintere Fläche (7) des optischen Bereichs (3) anschließt und einen Vorsprung in bezug auf die hintere Fläche (7) des optischen Bereichs (3) bildet, und durch eine vordere Fläche (10), die sich an die vordere Fläche (6) des optischen Bereichs (3) anschließt und zurückliegend in bezug auf die vordere Fläche (6) des optischen Bereichs (3) angeordnet ist, begrenzt ist,
dadurch gekennzeichnet, daß die jeweilige vordere (10) und hintere (11) Fläche des Verankerungsbereichs gegenseitig in dem Sinne einer Entfernung in bezug auf die optische Achse (5) aufeinander zu laufen, derart, daß eine fortschreitende Verdünnung des Verankerungsbereichs (4) beginnend von dem optischen Bereich (3) und bis zu einem Umfangsrand (12, 13) der Linse (1, 101) festgelegt ist, um es zu ermöglichen, den Verankerungsbereich (4) in einen ringförmigen Einschnitt (14, 114) der Hornhaut (2) einzusetzen.

2. Linse nach Anspruch 1, dadurch gekennzeichnet, daß in bezug auf eine Ebene, die rechtwinklig zur optischen Achse (5) liegt, die vordere Fläche (10) des Verankerungsbereichs (4) eine Neigung (B) zeigt, die deutlich kleiner als 90° ist.

3. Linse nach Anspruch 2, dadurch gekennzeichnet, daß, in bezug auf die Ebene, die senkrecht zu der optischen Achse (5) liegt, die hintere Fläche (11) des Verankerungsbereichs (4) eine Neigung (A) zeigt, die zwischen 40° und 45° liegt und insbesondere 42° beträgt, und die vordere Fläche (10) des Verankerungsbereichs (4) eine Neigung (B) zeigt, die zwischen 50° und 55° liegt.

4. Linse nach einem der Ansprüche 1 bis 3, dadurch gekennzeichnet, daß sie ausgehend von wenigstens einem Polymer hergestellt ist, das weich und biokompatibel ist, ausgewählt aus den natürlichen Polymeren und den künstlichen Polymeren.

5. Linse nach einem der Ansprüche 1 bis 4, dadurch gekennzeichnet, daß die vordere (10) und hintere (11) Fläche des Verankerungsbereichs (4) ungefähr dieselbe Form und dieselben Abmessungen (1) zeigen, wenn sie im Schnitt durch irgendeine Halbebene gesehen werden, die durch die optische Achse (5) begrenzt ist.

6. Linse nach einem der Ansprüche 1 bis 5, dadurch gekennzeichnet, daß die vordere (10) und hintere (11) Fläche des Verankerungsbereichs (4) ungefähr kegelstumpfförmig im Umlauf um die optische Achse (5) sind.

7. Linse nach einem der Ansprüche 1 bis 6, dadurch gekennzeichnet, daß die vordere (10) und hintere (11) Fläche des Verankerungsbereichs (4) sich gegenseitig vereinigen, an dem Umfangsrand (12) der Linse (1, 101) an einer kreisförmigen Kante (12).

8. Linse nach einem der Ansprüche 1 bis 6, dadurch gekennzeichnet, daß sich an die vordere (10) und hintere (11) Fläche des Verankerungsbereichs (4) jeweils, an dem Umfangsrand (112) der Linse (1, 101), eine Umfangsfläche (13) im Umlauf um die optische Achse (5) anschließt.

9. Linse nach Anspruch 8, dadurch gekennzeichnet, daß die Umfangsfläche (13) zylindrisch im Umlauf um die optische Achse (5) ist.

10. Linse nach einem der Ansprüche 1 bis 9, dadurch gekennzeichnet, daß die vordere Fläche (6) des optischen Bereichs (3) die Form einer kugelförmigen Kalotte zeigt, die auf der optischen Achse (5) zentriert ist.

11. Linse nach einem der Ansprüche 1 bis 10, dadurch gekennzeichnet, daß die vordere Fläche (10) des Verankerungsbereichs (4) sich direkt an die vordere Fläche (6) des optischen Bereichs (3) anschließt.

12. Linse nach einem der Ansprüche 1 bis 10, dadurch gekennzeichnet, daß die vordere Fläche (10) des Verankerungsbereichs (4) sich an die vordere Fläche (6) des optischen Bereichs (3) durch Zwischenschaltung eines peripheren Absatzes (8) desselben anschließt, der um die optische Achse (5) umläuft.

13. Linse nach Anspruch 12, dadurch gekennzeichnet, daß der periphere Absatz (8) zylindrisch im Umlauf um die optische Achse (5) ist.

14. Linse nach einem der Ansprüche 1 bis 13, dadurch gekennzeichnet, daß die hintere Fläche (7) des optischen Bereichs (3) die Form einer kugelförmigen Kalotte zeigt, die auf der optischen Achse (5) zentriert ist.

15. Linse nach einem der Ansprüche 1 bis 13, dadurch gekennzeichnet, daß die hintere Fläche (7) des optischen Bereichs (3) eine asphärische Form zeigt.

16. Linse nach Anspruch 15, dadurch gekennzeichnet, daß sie eine Markierung (21) in winkelmäßiger Ausrichtung um die optische Achse (5) zeigt.

17. Linse nach einem der Ansprüche 1 bis 16, dadurch gekennzeichnet, daß die hintere Fläche (11) des Verankerungsbereichs (4) sich direkt an die hintere Fläche (7) des optischen Bereichs (3) anschließt.

18. Linse nach einem der Ansprüche 1 bis 16, dadurch gekennzeichnet, daß die hintere Fläche (11) des Verankerungsbereichs (4) sich an die hintere Fläche (7) des optischen Bereichs (3) durch Zwischenschaltung einer peripheren Schulter (8) desselben anschließt, die um die optische Achse (5) umläuft.

19. Linse nach Anspruch 18, dadurch gekennzeichnet, daß die periphere Schulter (8) zylindrisch im Umlauf um die optische Achse (5) ist.

20. Linse nach einem der Ansprüche 1 bis 19, dadurch gekennzeichnet, daß der Verankerungsbereich (4) wenigstens eine Kerbe (21) zeigt, die in die Umfangskante (12, 13) der Linse (1, 101) und in Bereiche der vorderen (10) und hinteren (11) Fläche des Verankerungsbereichs (4) benachbart dem Umfangsrand (12, 13) der Linse (1, 101) mündet.

21. Keratotom, bestimmt zur Realisierung eines ringförmigen Einschnittes in eine Hornhaut und zu diesem Zweck aufweisend:
- einen äußeren rohrförmigen Träger (201), der eine Umhüllung bildet, welche eine festgelegte Achse (202) zeigt und für seine Anwendung bei einer Hornhaut ein Fußteil (204) aufweist, das ringförmig im Umlauf um die Achse (202) des Trägers (201) ist und eine geometrische Referenzfläche (210) begrenzt, die so wie die Form definiert ist, die die Hornhaut zeigt, wenn der Träger (201) auf dieselbe mit seinem Fußteil (204) angesetzt wird,
- einen inneren rohrförmigen Körper (203), der koaxial zum Inneren des Trägers (201) angeordnet ist, zum Inneren des Trägers (201) in bezug auf die Referenzfläche (210) zurückliegend,
- Einrichtungen (214, 218, 222, 224, 225) zum Führen des Körpers in seiner Drehung um die Achse (202) des Trägers (201) in bezug auf den Träger (201) um wenigstens 360°,
- Einrichtungen (232) zum Drehantreiben des Körpers (203) um die Achse (202) des Trägers (201) in bezug auf den Träger (201) um wenigstens 360°,
- ein Messer (268), das im Inneren des Körpers (203) angeordnet ist, einer charakteristischen Richtung (270) folgend, das in einem festgelegten Sinne (325) seiner charakteristischen Richtung (270) eine Schneidspitze (319) zeigt,
- Einrichtungen (269, 275, 308) zur Verbindung zwischen dem Messer (268) und dem Körper (203), die selbst Einrichtungen (308) zum Verlagern des Messers (268) in gesteuerter Weise in Verschiebebewegung entlang seiner charakteristischen Richtung (270), in bezug auf den Körper (203), zwischen einer Ruheposition, in der es zum Inneren des Körpers (203) hin in bezug auf die Referenzfläche (210) zurückliegend angeordnet ist und in der die Spitze (319) zu der Referenzfläche (210) gedreht ist, und einer Schneidposition, in der die Spitze (319) einen Vorsprung zum Äußeren des Körpers (203) in bezug auf die Referenzfläche (210) zeigt, versetzt in bezug auf die Achse (202) des Trägers (203), aufweisen,
dadurch gekennzeichnet, daß, im Hinblick auf das Realisieren eines Schnittes, der dazu bestimmt ist, den Verankerungsbereich einer Linse nach einem der Ansprüche 1 bis 20 aufzunehmen, die Einrichtungen (269, 275, 308) zur Verbindung zwischen dem Messer (268) und dem Körper (203) weiterhin Einrichtungen (275) zum gesteuerten Verlagern des Messers (268) zur Ausrichtung seiner charakteristischen Richtung (270) in bezug auf den Körper (203) aufweisen, zwischen mehreren bestimmten Ausrichtungen, mit einer ersten Ausrichtung (264), in der seine charakteristische Richtung (270) parallel zu der Achse (202) des Trägers (201) ist, und einer zweiten Ausrichtung (265), in der seine charakteristische Richtung (270) die Achse (202) des Trägers (201) schneidet und in bezug auf diese Achse (202) schräg ist, derart, daß wenigstens in der Schneidposition, die Spitze (319) des Messers (268) in dem genannten Sinne (325) in bezug auf die Achse (202) divergiert, wobei in den Ausrichtungen (264, 265) ein im wesentlichen identischer Abstand zwischen der Achse (202) des Trägers (201) und einem Bereich (262, 263), in dem die Spitze (319) des Messers (268) die Referenzfläche (210) in der Schnittposition durchquert, beibehalten wird.

22. Keratotom nach Anspruch 21, dadurch gekennzeichnet, daß die bestimmen Ausrichtungen (264, 265) an der Zahl zwei sind, nämlich die erste und zweite Ausrichtung (264, 265).

23. Keratotom nach Anspruch 22, dadurch gekennzeichnet, daß die Spitze (319) des Messers (268) die Referenzfläche (210), in der Schneidposition, in zwei Bereichen (262, 263) durchquert, die einander diametral in bezug auf die Achse (202) des Trägers (201) gegenüberliegen, jeweils in der einen und der anderen der genannten ersten und zweiten Ausrichtungen (264, 265).

24. Keratotom nach einem der Ansprüche 21 bis 23, dadurch gekennzeichnet, daß es im Inneren des Fußteils (204) eine Wand (253) aufweist, die einstückig mit dem Körper (203) ist und zum Außenraum des Körpers (203) hin eine Fläche zeigt, die mit der Referenzfläche (210) deckungsgleich ist, wobei die Wand (253) Einrichtungen (262, 263) für den Durchlaß des Messers (268) in der Schneidposition zeigt.

25. Keratotom nach Anspruch 24, dadurch gekennzeichnet, daß die Wand (253) Einrichtungen (259, 260) zum Schauen durch sie hindurch und/oder zum Positionsmarkieren in bezug auf die Hornhaut aufweist.

26. Keratotom nach Anspruch 23 und einem der Ansprüche 24 und 25, dadurch gekennzeichnet, daß die Durchlaßeinrichtungen (262, 263) zwei Löcher (262, 263) aufweisen.

27. Keratotom nach einem der Ansprüche 23 und 26, dadurch gekennzeichnet, daß die Einrichtungen (269, 265, 308) zur Verbindung zwischen dem Messer (268) und dem Körper (203)
- einen Messerkasten (269), der Einrichtungen (302, 308) zum Aufnehmen des Messers (268) und zum Führen desselben in der relativen Verschiebebewegung entlang der charakteristischen Richtung (270) aufweist und zum Inneren des Körpers (203) in bezug auf die Referenzfläche (210) zurückliegend angeordnet ist,
- Einrichtungen (273) zum Verschwenken des Messerkastens (269) auf dem Körper (203), um eine vorbestimmte Schwenkachse (272), die in bezug auf die Trägerachse (201) versetzt ist, zurückgezogen in das Innere des Körpers (203) in bezug auf die Referenzfläche (210) und senkrecht zu der charakteristischen Richtung (270) des Messers (268) und zu einer Ebene (261), die die Achse (202) des Trägers (201) einschließt,
- Einrichtungen (275), um in geregelter Weise den Messerkasten (269) um die Schwenkachse (272) schwenken zu lassen, in bezug auf den Körper (203) in die eine oder die andere der beiden Ausrichtungen (264, 265), die jeweils der ersten und zweiten Ausrichtung (264, 265) entspricht,
- Einrichtungen (308) zum Verlagern des Messers (268) in geregelter Weise in einer Verschiebebewegung in bezug auf den Messerkasten (269) zwischen entsprechenden Positionen für die eine oder die andere der Ausrichtungen (264, 265) des Messerkastens (269) in bezug auf den Körper (203), in eine Ruheposition und in eine Schneidposition
aufweist.

28. Keratotom nach Anspruch 27, dadurch gekennzeichnet, daß es Einrichtungen (288, 292, 293) zur Anlage des Messerkastens (269) in bezug auf den Körper (203) jeweils in der einen und/oder der anderen der genannten Ausrichtungen (264, 265) des Messerkastens (269) in bezug auf den Körper (203) aufweist.

29. Keratotom nach Anspruch 28, dadurch gekennzeichnet, daß die Anlageeinrichtungen (288, 292, 293) einstellbar sind.

30. Keratotom nach einem der Ansprüche 27 bis 29, dadurch gekennzeichnet, daß die Einrichtungen (308) zum Verlagern des Messers (268) einer Verschiebebewegung in geregelter Weise in bezug auf den Messerkasten (269) Einrichtungen (308) für die mikrometrische Einstellung sind.

31. Keratotom nach Anspruch 30, dadurch gekennzeichnet, daß die Einrichtungen (308) für die mikrometrische Einstellung auf dem Körper (203) um eine Achse (329) parallel zu der Schwenkachse (272) des Messerkastens (269) auf dem Körper (203) verschwenkt sind, entgegengesetzt zu der Referenzfläche (210) in bezug auf die Schwenkachse (272) des Messerkastens (269) auf dem Körper (203), und mit dem Messer (268) durch eine Schubkraft zusammenwirken, die auf dasselbe in dem bestimmten Sinne (325) seiner charakteristischen Richtung (270) aufgebracht wird, und daß der Messerkasten (269) Einrichtungen (326) für die elastische Belastung des Messers (268) im entgegengesetzten Sinne zu dem bestimmten Sinn (325) aufweist.

32. Keratotom nach einem der Ansprüche 21 bis 31, dadurch gekennzeichnet, daß es Indexiereinrichtungen für die winkelmäßige Position des Körpers (203) in bezug auf den Träger (201) um die Achse (202) des Trägers (201) aufweist.

33. Keratotom nach einem der Ansprüche 21 bis 32, dadurch gekennzeichnet, daß das Fußteil (204) einen ringförmigen Hohlraum (206) begrenzt, der um die Referenzfläche (210) offen ist, und daß Einrichtungen (213) vorgesehen sind, um den ringförmigen Hohlraum (206) in Unterdruck zu bringen.
